(19) European Patent Office
Office européen des brevets

(11) Publication number: **0 122 155 A2**

# EUROPEAN PATENT APPLICATION

(21) Application number: **84302457.1**

(22) Date of filing: **11.04.84**

(51) Int. Cl.³: **C 07 D 501/20**, A 61 K 31/545

(30) Priority: **12.04.83 US 484128**
**30.09.83 US 548050**
**22.07.83 US 516220**

(43) Date of publication of application: **17.10.84**
**Bulletin 84/42**

(84) Designated Contracting States: **AT BE CH DE FR GB IT LI LU NL SE**

(71) Applicant: **ELI LILLY AND COMPANY, 307, East McCarty Street, Indianapolis Indiana 46285 (US)**

(72) Inventor: **Cooper, Robin David Grey, 6740, Dover Road, Indianapolis Indiana 46220 (US)**
Inventor: **Blaszczak, Larry Chris, 5825 North Broadway, Indianapolis Indiana 46220 (US)**
Inventor: **Turner, Jan Ross, 651 Ash Drive, Carmel Indiana 46032 (US)**
Inventor: **Conrad, Preston Charles, 7632 Teel Way, Indianapolis Indiana 46256 (US)**
Inventor: **Daugherly, Byron Wade, 2305, Brewer Drive, Indianapolis Indiana 46227 (US)**

(74) Representative: **Crowther, Terence Roger et al, Erl Wood Manor, Windlesham Surrey GU20 6PH (GB)**

(54) **Improvements in or relating to naphthylglycyl cephalosporin derivatives.**

(57) Naphthylglycyl and tetrahydronaphthylglycyl cephalosporins are potent antibacterial agents and particularly useful in the oral treatment of upper respiratory infections.

EP 0 122 155 A2

# IMPROVEMENTS IN OR RELATING TO NAPHTHYLGLYCYL CEPHALOSPORIN DERIVATIVES

This invention relates to a new class of cephalosporins which are orally-effective and possess advantageous pharmacokinetic properties.

The cephalosporin antibiotics have been studied extensively, and several members of this class are used routinely to combat bacterial diseases caused by a broad spectrum of gram positive and gram negative microorganisms. The majority of such compounds are not effective orally, but rather are administered intramuscularly or intravenously, necessitating assistance from medically-trained personnel. Moreover, because the compounds are effective against a broad spectrum of microorganisms, they generally are not employed for their specificity.

A need remains for cepahlosporin antibiotics that are orally effective and have a degree of specificity toward one or more groups of microorganisms. This invention provides a group of compounds that satisfy these needs.

In accordance with the invention, a 2-naphthylglycylamido cephalosporin of Formula (I):

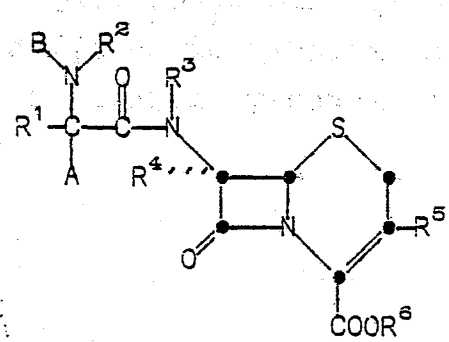

(I)

in which

$R^1$ is

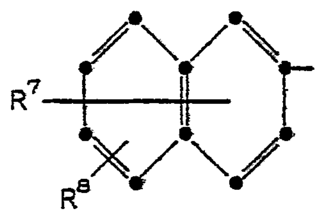 , 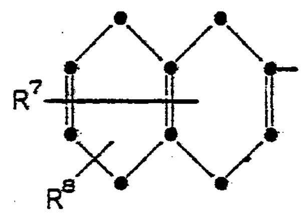 , or

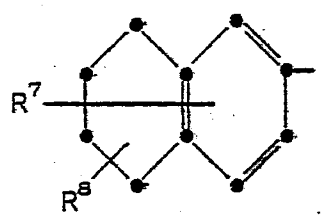 .

in which $R^7$ and $R^8$, independently, are hydrogen, halo, hydroxy, $C_1$-$C_4$ alkyl, $C_1$-$C_4$ alkoxy, nitro, amino, $C_1$-$C_4$ alkanoylamino, $C_1$-$C_4$ alkylsulfonylamino, or when $R^7$ and $R^8$ are taken together they form methylenedioxy;

A and B, both, are hydrogen, or taken together complete a double bond;

$R^2$ is hydrogen, an amino protecting group, hydroxy, or methoxy, and $R^3$ is hydrogen, or $R^2$ and $R^3$ taken together are

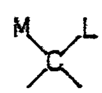 ,

where M and L independently, are $C_1$-$C_4$ alkyl;

$R^4$ is hydrogen, methoxy or methylthio;

$R^5$ is hydrogen, methoxy, methyl, halo, or methoxymethyl;

$R^6$ is hydrogen, or a carboxy-protecting group; with the proviso that $R^2$ is hydroxy or methoxy only when A and B complete a double bond, and that A and B, both, are hydrogen when $R^3$ is other than hydrogen; or a pharmaceutically-acceptable salt thereof, is useful as an orally-active antibiotic or as intermediates thereto.

Preferred compounds provided by the invention include those of Formula (I) in which

$R^1$ is

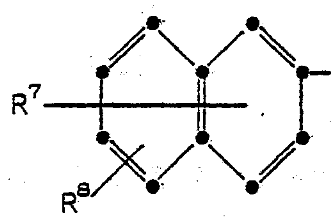

and $R^7$ and $R^8$ are as defined previously. Within this group, preferred compounds include those in which $R^2$ is hydrogen, an amino-protecting group, hydroxy or methoxy, and $R^6$ is hydrogen or a carboxy-protecting group.

Another preferred group of compounds are those in which $R^1$ is

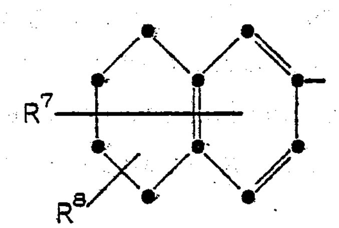

and $R^7$ and $R^8$ are as defined previously. Especially preferred compounds within this group include those in which A, B, $R^2$, $R^3$, $R^4$ and $R^6$ are all hydrogen.

A particularly preferred group of compounds of this invention is defined by the Formula:

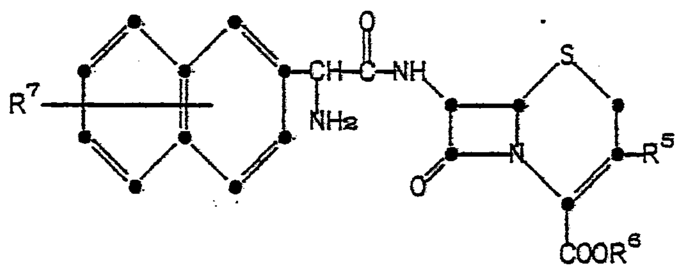

in which $R^5$, $R^6$ and $R^7$ are as defined previously. The most preferred compounds are those in which $R^7$ is hydrogen, halo, hydroxy or methoxy, $R^5$ is methyl or chloro, and $R^6$ is hydrogen, or a pharmaceutically-acceptable salt thereof, such as the sodium or potassium salt.

This invention also provides a pharmaceutical formulation comprising a naphthylglycylamido cephalosporin derivative of Formula (I), or a pharmaceutically-acceptable salt thereof, associated with a pharmaceutical carrier, diluent or excipient therefor. A preferred formulation is one suitable for oral administration.

In another embodiment of this invention there is provided a method for treating bacterial infections in animals which comprises administering to the animal an effective amount of an antibacterial compound of

Formula (I), or a pharmaceutically-acceptable salt thereof. In a preferred method of treatment, the naphthylglycylamido cephalosporin derivative is administered orally to treat diseases caused by gram positive bacteria.

In addition, there is provided a process for preparing a compound of Formula (I) which comprises

(A) acylating a compound of Formula (II):

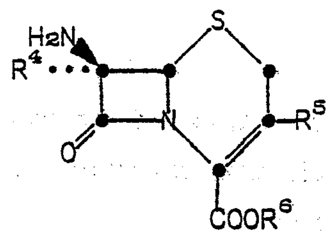

(II)

with an acylating agent of Formula (III):

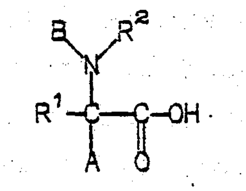

(III),

or an activated derivative thereof, in which A, B, $R^1$, $R^2$, $R^4$, $R^5$ and $R^6$ are as defined above, followed, optionally, by removal of any amino- or carboxyl-protecting groups present;

(B) deblocking of a protected acid of Formula (I) in which $R^6$ is a carboxy-protecting group to provide a compound of Formula (I) in which $R^6$ is hydrogen;

(C) removal of an amino-protecting group, $R^2$, from a compound of Formula (I) to provide a compound of Formula (I) in which $R^2$ is hydrogen;

(D) when it is desired to form a compound in which $R^2$ and $R^3$, when taken together form a group of the Formula:

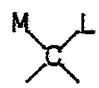 ,

reacting a compound of Formula (I), in which $R^2$ and $R^3$ both are hydrogen, with a ketone of the Formula:

$$M-C(O)-L$$

in which M and L are as defined earlier; or,

(E) reducing a compound of Formula (I) in which A and B are taken together to form a double bond and $R^2$ is hydroxy or methoxy, to produce a compound of Formula (I) in which A, B, and $R^2$ are hydrogen; and,

(F) if desired, salifying a compound of formula (I),

(G) or, if desired, converting a salt of a compound of Formula (I) to the free amine or acid.

In the formulae above, $R^1$ represents a 2-naphthyl or 2-tetrahydronaphthyl group. The naphthyl and tetrahydronaphthyl groups may be unsubstituted, for instance, when $R^7$ and $R^8$ both are hydrogen; mono-substituted at the 1, 3, 4, 5, 6, 7 or 8 positions, for

instance, when $R^8$ is hydrogen and $R^7$ is other than hydrogen; or di-substituted when $R^7$ and $R^8$, both, are other than hydrogen. The groups with which the naphthyl and tetrahydronaphthyl rings may be substituted include hydroxy, $C_1$-$C_4$ alkyl, $C_1$-$C_4$ alkoxy, halo, nitro, amino, $C_1$-$C_4$ alkanoylamino and $C_1$-$C_4$ alkylsulfonylamino.

The term "$C_1$-$C_4$alkyl" carries its art-recognized meaning of straight and branched lower alkyl carbon chains such as methyl, ethyl, _iso_propyl, _n_-propyl, _iso_-butyl and _tert_-butyl. Similarly, "$C_1$-$C_4$ alkoxy" refers to lower alkyl groups bonded to the naphthyl ring through an oxygen atom. Typical $C_1$-$C_4$ alkoxy groups include methoxy, ethoxy, _n_-propoxy, _n_-butoxy and _iso_-butoxy. The term "halo" includes fluoro, chloro, bromo and iodo. A preferred halo group is chloro. "$C_1$-$C_4$ Alkanoylamino" refers to an acyl residue of a lower alkanoic acid, bonded to the naphthyl or tetrahydronaphthyl ring through a nitrogen atom. Such groups include formylamino, acetylamino and butyrylamino. "$C_1$-$C_4$ Alkylsulfonylamino" means a group such as methylsulfonylamino, ethylsulfonylamino and n-butylsulfonylamino.

$R^2$ represents a substituent on the glycyl nitrogen atom, and may include hydrogen and an amino-protecting group. The term "amino-protecting group" refers to any of the art-recognized substituents that can be attached to an amino nitrogen atom and which is removed readily when desired. Such protecting groups often are employed during preparation of the compounds of the invention, and serve to decrease the likelihood of unwanted side reactions occurring as a result of the presence of a free amino group. While the compounds in

which $R^2$ is a protecting group will have biological activity, it is contemplated that the most biologically desirable compounds will be those in which $R^2$ is hydrogen. The compounds in which $R^2$ is an amino-protecting group are thus primarly useful as intermediates in the synthesis of the more preferred free amino compounds.

The precise nature of the amino-protecting group is not critical to the invention, and any of the well-known protecting groups can be employed. Typical amino-protecting groups are described by J.W. Barton in "Protective Groups in Organic Chemistry," J.F. McOmie, Ed., Plenum Press, New York, N.Y., 1973, Chapter 2, and by Greene in "Protective Groups in Organic Synthesis", John Wiley & Sons, New York, N.Y., 1981, Chapter 7. Both of these references are incorporated herein by reference for their teaching of amino-protecting groups.

The most common amino-protecting groups to be employed may include $C_1$-$C_{10}$ alkanoyl and halo $C_1$-$C_{10}$ alkanoyl groups such as formyl, acetyl, chloroacetyl, dichloroacetyl, propionyl, hexanoyl, 3,3-diethylhexanoyl, or γ-chlorobutyryl; $C_1$-$C_{10}$ alkoxycarbonyl and $C_2$-$C_{10}$ alkenyloxycarbonyl groups such as methoxycarbonyl, tert-butoxycarbonyl, and allyloxycarbonyl; $C_5$-$C_{15}$ aryloxycarbonyl and arylalkenyloxycarbonyl such as benzyloxycarbonyl, 4-nitrobenzyloxycarbonyl and cinnamoyloxycarbonyl; halo-$C_1$-$C_{10}$ alkoxycarbonyl such as 2,2,2-trichloroethoxycarbonyl; and $C_5$-$C_{15}$-arylalkyl and alkenyl groups such as benzyl, phenethyl, trityl, or allyl.

Other commonly used amino-protecting groups include enamines prepared by reaction of the free amino

compound with a β-keto-ester such as methyl or ethyl acetoacetate.

In addition to representing hydrogen or an amino-protecting group, $R^2$ in the above formulae can be taken with $R^3$ to complete a ring system so as to form compounds of the Formula:

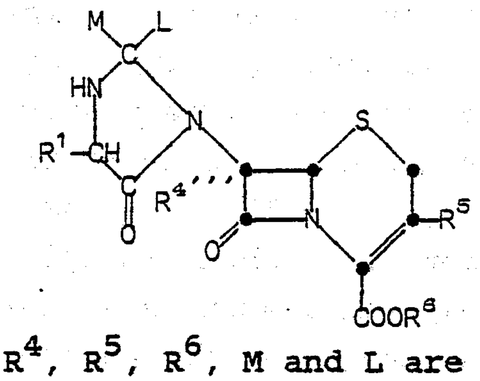

in which $R^1$, $R^4$, $R^5$, $R^6$, M and L are as defined previously. Exemplary of such compounds are the acetonides, those in which M and L are both methyl. Such compounds are prepared by reacting a glycylamido cephalosporin in which $R^2$ and $R^3$, both, are hydrogen with a ketone such as acetone. These cyclic compounds are particularly useful as long-acting antibacterial agents.

$R^6$ in the above formula is hydrogen, an addition salt cation such as ammonium or an alkali metal cation such as lithium, sodium or potassium; or a carboxy-protecting group. The term "carboxy-protecting group" refers to the art-recognized groups commonly employed to block or protect the carboxylic acid functionality of a cephalosporin during chemical reactions involving other functional sites in the molecule, and which can be removed readily when desired by commonly known techniques such as hydrolysis or hydrogenolysis.

Typical carboxy-protecting groups to be employed according to this invention include those described by E. Haslam in "Protective Groups in Organic Chemistry," supra, Chapter 5, and by Greene in "Protective Groups in Organic Synthesis," supra, Chapter 5, which are incorporated herein by reference. Examples of commonly employed carboxy-protecting groups may include $C_1$-$C_{10}$ alkyl groups such as methyl, tert-butyl, decyl; halo-$C_1$-$C_{10}$ alkyl such as 2,2,2-trichloroethyl, and 2-iodoethyl; $C_5$-$C_{15}$ arylalkyl such as benzyl, 4-methoxybenzyl, 4-nitrobenzyl, diphenylmethyl, triphenylmethyl; $C_1$-$C_{10}$ alkanoyloxymethyl such as acetoxymethyl, or propionoxymethyl; and other groups such as phenacyl, 4-halophenacyl, allyl, dimethylallyl, tri($C_1$-$C_3$ alkyl)-silyl such as trimethylsilyl, and related groups.

The naphthylglycyl and tetrahydronaphthylglycyl cephalosporins provided by this invention can be prepared by any of several methods, one of which comprises coupling a 7-aminocephalosporin nucleus of Formula (II):

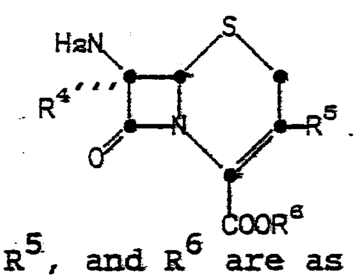

(II)

in which $R^4$, $R^5$, and $R^6$ are as defined previously, to a naphthylglycine compound of Formula (III), or an activated derivative thereof:

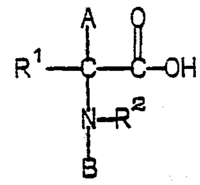

(III)

in which A, B, $R^1$, $R^2$, $R^4$, $R^5$ and $R^6$ are as defined earlier. Examples of cephalosporin nuclei that may be employed in the synthesis of the compounds of this invention may include those in which $R^4$, $R^5$ and $R^6$ have the following meanings:

| $R^4$ | $R^5$ | $R^6$ |
|---|---|---|
| H | $-CH_3$ | H |
| H | $-CH_3$ | tert-butyl |
| $CH_3O-$ | $-CH_3$ | p-nitrobenzyl |
| H | $-CH_3$ | 2,2,2-trichloroethyl |
| H | -Cl | H |
| H | -Cl | tert-butyl |
| H | -Cl | phenacyl |
| $CH_3S$ | -Cl | methyl |
| $CH_3O$ | H | H |
| H | H | p-nitrobenzyl |
| $CH_3S$ | $-CH_2OCH_3$ | diphenylmethyl |
| H | $-CH_2OCH_3$ | tert-butyl |
| H | H | trimethylsilyl |
| H | -Cl | trityl |
| $CH_3O-$ | $-CH_3$ | tert-butyl |
| H | -Br | H |
| H | -F | methyl |
| H | $-OCH_3$ | H |
| H | $-CH_3$ | p-nitrobenzyl |
| $CH_3S-$ | $-CH_3$ | tert-butyl |
| H | -Cl | allyl |
| H | -Br | 2,2,2-trichloroethyl |
| H | -I | methyl |

The 7-aminocephalosporin nuclei to be employed in the synthesis of compounds of this invention are well-known in the art and are readily available by art known methods. For example, the 3-halo cephalosporin nuclei are available by the methods described in U.S. Patent No. 3,925,372. 3-Methyl cephalosporin nuclei can be prepared by ring expansion of penicillin sulfoxides and subsequent side chain cleavage, or by hydrogenation of 3-acetoxymethyl derivatives.

The term "activated derivative" means a derivative which renders the carboxyl function of the acylating agent of Formula (III) reactive to coupling with a primary amino group to form the amide bond which links the acyl side chain to the nucleus. Suitable activated derivatives, their methods of preparation, and their use as acylating agents for a primary amine will be recognized by those skilled in the art. Preferred activated derivatives are: (a) an acid halide such as the chloride or bromide, or (b) alkanoyloxy derivatives such as formyloxy or acetoxy mixed anhydrides (e.g. Y in the list below is HCHO or $OCOCH_3$). Other methods for activating the carboxyl function may include reaction of the carboxylic acid with a carbodiimide (e.g. N,N'-dicyclohexylcarbodiimide or N,N'-diisopropylcarbodiimide) to give a reactive intermediate which is reacted *in situ* with the 7-amino group. This reaction is discussed in detail later.

Similarly, the naphthylglycyl and tetrahydronaphthylglycyl reactants defined by Formula (III) are known in the art and are prepared employing well-known procedures. Typical naphthylglycyl and tetrahydronaphthylglycyl derivatives which may be employed to prepare the compounds of the invention have the above formula in which $R^2$, $R^7$, and $R^8$ may have the following meanings:

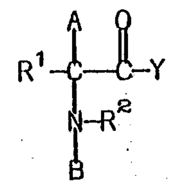

| $R^7$ | $R^8$ | $R^2$ | Y |
|---|---|---|---|
| H | H | H | Cl (hydrochloride) |
| H | H | chloroacetyl | OH |
| H | 5-$OCH_3$ | allyloxycarbonyl | OH |
| H | 6-$OCH_3$ | *tert*-butoxycarbonyl | Cl |
| H | 7-$OCH_3$ | benzyl | Br |
| H | 8-$OCH_2CH_3$ | trimethylsilyl | OCHO |
| 1-Cl | 5-$OCH_3$ | p-nitrobenzyl | $OCOCH_3$ |
| 3-Cl | 7-Cl | H | Br (hydrobromide) |
| 3-Cl | H | benzyloxycarbonyl | Br |
| 4-$OCH_3$ | | *tert*-butoxycarbonyl | Cl |
| H | 7-$NO_2$ | methyl acetoacetate enamine | OH |
| 3-$NO_2$ | 8-I | 2,2,2-trichloro ethoxycarbonyl | Cl |
| 7-$CH_2CH_2$ | 5-$CH_3$ | allyloxycarbonyl | OH |
| H | 6-$CH_3$ | formyl | Cl |
| H | 7-$CH_2CH_3$ | acetyl | OH |
| 1-OH | 8-$CH_2CH_2CH_3$ | benzoyl | HCHO |

The coupling of a naphthylglycine or tetrahydronaphthylglycine derivative with a 7-aminocephalosporin nucleus can be accomplished employing common techniques of acylation. For example, a naphthylglycyl acylating agent, especially the acid chloride or bromide or an alkanoyloxy derivative such as formyloxy or acetoxy anhydrides, can be reacted with a cephalosporin nucleus employing standard acylation conditions. During such acylation reactions, it generally is preferred that $R^2$ be an amino-protecting group and that $R^6$ be a carboxy-protecting group. These protecting groups serve to minimize unwanted side reactions and to increase solubility characteristics of the respective reactants.

The acylation reaction generally is accomplished by combining approximately equimolar quantities of a naphthylglycyl or tetrahydronaphthylglycyl acylating agent of Formula (III) (e.g. an acid halide or mixed acid anhydride) with the 7-aminocephalosporin nucleus. The acylation reaction normally is performed in a mutual solvent such as benzene, chloroform, dichloromethane, toluene, N,N-dimethylformamide, or acetonitrile, and routinely is complete after about 1 to about 12 hours when conducted at a temperature of about -20 to about 60°C. About an equimolar quantity of a base such as pyridine, triethylamine, aniline, or sodium carbonate can be employed in the reaction, if desired, to act as an acid scavenger. The product may be isolated from the reaction mixture by removing the reaction solvent, for instance by evaporation under reduced pressure, and further purification can be accomplished, if needed, employing routine techniques

such as chromatography, crystallization, solvent extraction, and other related methods.

An alternative and preferred method for coupling a naphthylglycyl or tetrahydronaphthylglycyl derivative to a 7-aminocephalosporin nucleus to produce compounds of the invention employs a coupling reagent such as those routinely used in the synthesis of peptides. Typical coupling reagents that may be employed include carbodiimides such as N,N'-diethylcarbodiimide, N,N-diisopropylcarbodiimide, and N,N-dicyclohexylcarbodiimide (DCC); carbonyl coupling reagents such as carbonyldiimidazole; isoxazolinium salts such as N-ethyl-5'-phenylisoxazolinium-3'-sulfonate; and quinoline compounds such as N-ethoxycarbonyl-2-ethoxy-1,2-dihydroquinoline (EEDQ).

The coupling of a 7-aminocephalosporin nucleus with a naphthylglycyl or tetrahydronaphthylglycyl derivative employing a peptide coupling reagent generally is accomplished by combining approximately equimolar quantities of a 7-aminoceph-3-em-4-carboxylic acid derivative, a naphthylglycine derivative, and a peptide coupling reagent according to the following scheme:

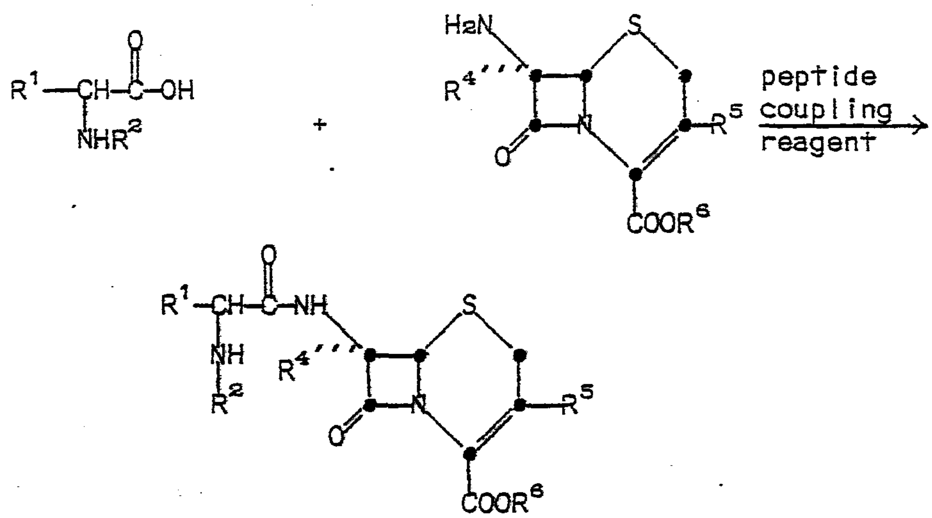


in which $R^1$, $R^2$, $R^4$, $R^5$ and $R^6$ are as defined earlier. Preferably, during such coupling reactions, $R^2$ is an amino-protecting group and $R^6$ is hydrogen or a carboxy-protecting group. Any protecting groups can be removed subsequently by standard methods to give the active antibiotic of the invention.

The coupling reaction normally is conducted in a mutual solvent such as dichloromethane, acetone, water, acetonitrile, N,N-dimethylformamide, or chloroform, and routinely is complete when carried out for about ten to about ninety minutes at a temperature of about -20 to about 60°C. Longer reaction periods are not detrimental and can be employed if desired. The product, a naphthylglycyl or tetrahydronaphthylglycyl cephalosporin, is isolated readily by simply removing the reaction solvent, for instance by evaporation under reduced pressure. The product may be purified further

by other standard methods such as acid-base extraction, chromatography, salt formation or the like.

Another method for preparing compounds of the invention employs a naphthyl oxime of the formula

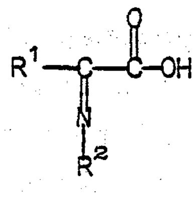

,

or an activated derivative thereof, in which $R^1$ is defined above, and $R^2$ is hydroxy or methoxy. When $R^2$ is hydroxy, it typically will be protected with a group such as trimethylsilyl or similar hydroxy protecting group. Such naphthyl oxime derivatives can be coupled to a cephalosporin nucleus by any of the methods described above to provide a compound of the Formula:

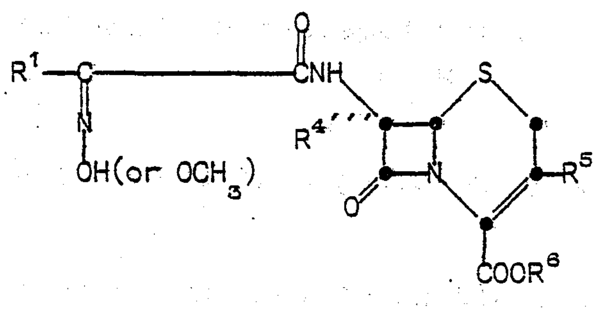

in which $R^1$, $R^4$, $R^5$, and $R^6$ are as defined earlier. These compounds are useful as intermediates because they are reduced readily by normal methods to give the preferred naphthylglycylamido compounds of the invention.

Additionally, the oximes of the above formula in which $R^6$ is hydrogen, or a salt thereof, are useful as antibiotics.

Compounds that bear a nitro group on the naphthylglycyl or the tetrahydronaphthylglycyl side chain can be modified to provide other compounds of the invention. For example, the nitro substituent can be reduced by routine reduction or hydrogenation procedures to give the corresponding amino substituted naphthylglycyl cephalosporin derivative, and if desired the amino group can be acylated by reaction with a $C_1$-$C_4$ alkanoyl halide or anhydride or a $C_1$-$C_4$ alkylsulfonyl halide to provide the corresponding alkanoylamino or alkylsulfonylamino naphthylglycylamido and tetrahydronaphthylglycylamido cephalosporins of the invention.

Similarly, compounds of the invention in which $R^2$ and $R^3$ are taken together to form the group

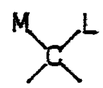

are prepared by reacting a ketone of the formula $M-\overset{\overset{O}{\|}}{C}-L$ with a compound of the invention in which $R^2$ and $R^3$, both, are hydrogen, generally in the presence of an acid such as methanesulfonic acid or the like. The cyclic compounds produced, for instance the preferred acetonides in which M and L both are methyl, are particularly useful as oral antibiotics because they are effective over prolonged periods of time.

Other compounds of the invention that are expected to be particularly long acting antibiotics are those in which $R^2$ is an alkanoyl amino-protecting group such as formyl or acetyl. Such compounds are prepared conveniently by simply reacting a naphthylglycylamido cephalosporin, in which $R^2$ is hydrogen, with a $C_1$-$C_{10}$ alkanoyl acylating agent, for instance formyl chloride or acetic anhydride. These N-acylated products are expected to act not only as antibiotics in themselves, but also as pro-drugs in that they will be hydrolyzed in an animal system to the parent naphthylglycyl or tetrahydronaphthylglycyl derivative.

Because the naphthylglycyl and tetrahydronaphthylglycyl side chains of the cephalosporins of this invention contain an asymmetric carbon atom, for example when A is hydrogen, the compounds of the invention can exist in the form of optical isomers, namely the D and the L isomers. The compounds of the invention can be employed as a D,L-mixture to treat bacterial infections in animals, or if desired the optical isomers can be separated and employed individually. While both isomers are effective antibacterial agents, one isomer appears to be more potent than the other and is designated the D-isomer, and accordingly is a preferred embodiment of the invention.

Separation or resolution of the optical isomers can be accomplished by routine methods carried out on the cephalosporin product of the invention or on the naphthylglycine or tetrahydronaphthylglycine side chain employed as a starting material. Separation of optical isomers generally will be accomplished by high

performance chromatography, enzymatic resolution, or chemical crystallization or racemization. A particularly preferred method for obtaining D-naphthylglycine comprises reacting the D,L-mixture with benzaldehyde and optically active tartaric acid according to the method of U.S. Patent No. 3,976,680.

As noted above, preferred compounds of the invention are those in which $R^2$ in the above formula is hydrogen. Such compounds, being primary amines, are basic in nature and readily form pharmaceutically-acceptable salts by reaction with acids. Those salts which are pharmaceutically-acceptable are preferred forms of salts used to treat bacterial infections. "Pharmaceutically-acceptable" salts are those salts useful in the chemotherapy of warm-blooded animals. Typical acids commonly employed to form salts include inorganic acids such as hydrogen chloride, hydrogen bromide, sulfuric acid, or phosphoric acid; as well as organic acids such as acetic acid, trifluoroacetic acid, succinic acid, methanesulfonic acid, oxalic acid, or para-toluenesulfonic acid. The compounds of the invention in which both $R^2$ and $R^6$ are hydrogen readily form an internal acid addition salt, namely a zwitterion.

The compounds of the invention generally exist as crystalline solids and can be crystallized from common solvents such as ethanol, water, N,N-dimethylformamide, acetone and the like. Further, the compounds in which $R^6$ is hydrogen are the 4-carboxylic acids. Such compounds are acidic and readily form salts with organic and inorganic bases. "Pharmaceutically-acceptable salts", as used herein, also includes these base addition salts.

The compounds of the present invention often crystallize as a solvate or hydrate and can be used in this form. As an example, this invention provides a crystalline composition of matter which is 7-(D-2-naphthylglycylamido)-3-methyl-3-cephem-4-carboxylic acid tetrahydrate. The crystals are large, dense and stable, and readily lend themselves to milling and grinding for adaptation to pharmaceutical formulation, particularily into solid dosage forms such as filled capsules and the like. The tetrahydrate of this invention is prepared by isolating 7-(D-2-naphthylglycylamido)-3-methyl-3-cephem-4-carboxylic acid, a preferred product of the invention, from an aqueous medium.

This crystalline compound has the following unique x-ray powder diffraction properties when measured with a 114.6 mm Debye-Scherrer camera containing a nickel filtered copper radiation of 1.5405Å:

| Spacing, d: | Relative intensities, $I/I_1$ |
|---|---|
| 13.29 | .44 |
| 10.53 | .22 |
| 7.14 | .44 |
| 6.58 | 1.00 |
| 6.09 | .22 |
| 5.64 | .33 |
| 5.36 | .88 |
| 5.14 | .28 |
| 4.86 | .17 |
| 4.66 | .11 |
| 4.44 | .33 |
| 4.12 | .28 |
| 3.92 | .17 |
| 3.75 | .22 |
| 3.65 | .94 |
| 3.51 | .38 |
| 3.34 | .72 |
| 2.94 | .39 |
| 2.78 | .17 |
| 2.69 | .14 |
| 2.58 | .11 |

| | |
|---|---|
| 2.46 | .17 |
| 2.42 | .17 |
| 2.32 | .11 |
| 2.25 | .03 |
| 2.18 | .14 |
| 2.10 | .06 |
| 2.04 | .06 |

In particular, this compound can be prepared by reacting an acid addition salt of 7-(D-2-naphthylglycylamido)-3-methyl-3-cephem-4-carboxylic acid with a base such as sodium hydroxide or triethylamine so as to form the corresponding zwitterion, and crystallizing the zwitterion from water. For example, a salt such as the trifluoroacetic acid salt or the hydrochloric acid salt can be dissolved in water or a mixture of water and an organic solvent such as acetone or acetonitrile. A base such as aqueous ammonium hydroxide is added to adjust the pH to about 3 to about 5. The precipitate that forms is the tetrahydrate of this invention and is readily recrystallized from water.

The compound of this invention alternatively can be prepared by isolating the product of the acylation of 7-amino-3-methyl-3-cephem-4-carboxylic acid (7-ADCA) from a solvent containing water. For example, 7-ADCA, typically as a silylated derivative, may be acylated with an N-protected D-2-naphthylglycine acid halide or mixed anhydride as described earlier. The acylation

generally may be carried out in an organic solvent such as acetonitrile. Once the acylation is complete, the protecting groups can be removed by standard procedures and the solution of 7-(D-2-naphthylglycylamido)-3-methyl-3-cephem-4-carboxylic acid can be diluted with water so that it contains about 10 to 50% by volume of water, and the pH of the solution can be adjusted to about pH 3 to about 5. The crystalline product produced is 7-(D-2-naphthylglycylamido)-3-methyl-3-cephem-4-carboxylic acid tetrahydrate.

This crystal form is very stable for prolonged periods of time, yet is very well absorbed following oral administration. This is somewhat surprising since the compound is only minimally soluble in water. For example, the compound forms a saturated solution in water at 37°C according to the following table:

| pH | Solubility, mg/ml |
|---|---|
| 1.2 | 8.6 |
| 2.0 | 1.7 |
| 3.0 | 0.2 |
| 4.0 | 0.2 |
| 5.0 | 0.2 |
| 6.0 | 0.3 |
| 7.0 | 0.8 |
| 7.5 | 1.2 |
| 8.0 | 4.1 |
| 8.5 | 7.5 |

Another example of a hydrated form of a compound of the invention is 7-(D-2-naphthylglycylamido)-3-methyl-3-cephem-4-carboxylic acid hydrochloride monohydrate.

The crystals of this compound are large, dense and stable, and readily lend themselves to milling and grinding for adaptation to pharmaceutical formulation, particularily into solid dosage forms such as filled capsules and the like. The hydrochloride monohydrate is prepared by crystallizing 7-(D-2-naphthylglycylamido)-3-methyl-3-cephem-4-carboxylic acid from an aqueous hydrochloric acid medium.

The crystalline hydrochloride monohydrate has the following unique x-ray powder diffraction properties when measured with a 114.6 mm Debye-Scherrer camera containing a nickel filtered copper radiation of 1.5405Å:

| Spacing, d: | Relative intensities, $I/I_1$ |
|---|---|
| 15.64 | 1.00 |
| 7.90 | .57 |
| 6.61 | .24 |
| 6.22 | .05 |
| 5.74 | .24 |
| 5.54 | .12 |
| 5.11 | .29 |
| 4.75 | .10 |
| 4.47 | .14 |
| 4.23 | .19 |
| 4.10 | .17 |
| 3.93 | .71 |
| 3.85 | .12 |
| 3.71 | .33 |
| 3.49 | .07 |
| 3.35 | .12 |
| 3.27 | .14 |
| 3.15 | .10 |
| 3.08 | .07 |
| 3.03 | .07 |
| 2.93 | .10 |
| 2.78 | .10 |
| 2.69 | .12 |
| 2.61 | .02 |
| 2.53 | .10 |
| 2.47 | .10 |
| 2.37 | .10 |
| 2.30 | .02 |
| 2.24 | .02 |
| 2.17 | .05 |

The hydrochloride monohydrate provided by this invention can be prepared by reacting 7-(D-2-naphthylglycylamido)-3-methyl-3-cephem-4-carboxylic acid, generally as a solvate or as the crystalline tetrahydrate described earlier, with hydrochloric acid in water or a solution of water and an organic solvent such as acetone or acetonitrile. Sufficient hydrochloric acid is employed to maintain the pH of the solution below about 2.0, generally about 0.1 to about 0.8. The reaction normally is complete after about one to about three hours when carried out at a temperature of about 0 to about 60°C. The precipitate that forms is the hydrochloride monohydrate and is isolated readily by standard means.

Alternatively, the compound can be prepared by isolating the product of the acylation of 7-amino-3-methyl-3-cephem-4-carboxylic acid (7-ADCA) from a solvent containing aqueous hydrochloric acid. For example, 7-ADCA, typically as a silylated derivative, can be acylated with an N-protected D-2-naphthylglycine acid halide or mixed anhydride. The acylation generally may be carried out in an organic solvent such as acetonitrile. Once the acylation is complete, the protecting groups can be removed by standard procedures and the solution of 7-(D-2-naphthylglycylamido)-3-methyl-3-cephem-4-carboxylic acid can be diluted with water so that it contains about 10 to 50% by volume of water, and the pH of the solution can be adjusted to about pH 0.1 to about 0.8 by addition of hydrochloric acid. The crystalline product that forms is 7-(D-2-naphthylglycylamido)-3-methyl-3-cephem-4-carboxylic acid hydrochloride monohydrate.

Examples of typical classes of naphthylglycyl and tetrahydronaphthylglycyl cephalosporins, as well as specific compounds provided by this invention, include those listed below:

A. Preferred compounds of the Formula:

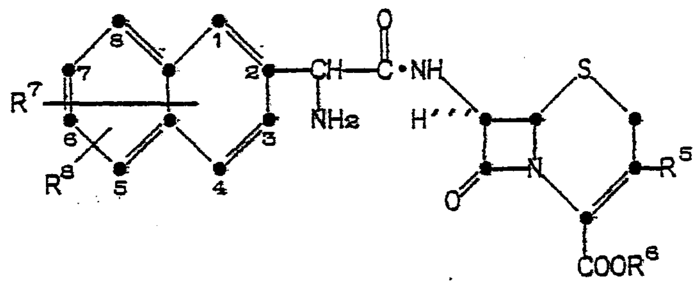

1. $R^6$ is hydrogen or a salt cation;

a. $R^7$ and $R^8$ both are hydrogen;

1a1. $R^5$ is methyl;
1a2. $R^5$ is chloro;
1a3. $R^5$ is bromo;
1a4. $R^5$ is fluoro;
1a5. $R^5$ is iodo;
1a6. $R^5$ is hydrogen;
1a7. $R^5$ is methoxy;
1a8. $R^5$ is methoxymethyl;

b. $R^7$ is hydrogen and $R^8$ is 6-methoxy;

1b1. $R^5$ is methyl;
1b2. $R^5$ is chloro;
1b3. $R^5$ is methoxy;
1b4. $R^5$ is methoxymethyl;

c. $R^7$ is hydrogen and $R^8$ is 7-fluoro;

1c1. $R^5$ is methyl;

1c2. $R^5$ is chloro;

1c3. $R^5$ is fluoro;

1c4. $R^5$ is methoxy;

1c5. $R^5$ is methoxymethyl;

d. $R^7$ is hydrogen and $R^8$ is 6-hydroxy;

1d1. $R^5$ is methyl;

1d2. $R^5$ is chloro;

1d3. $R^5$ is methoxy;

1d4. $R^5$ is bromo;

e. $R^7$ is 4-methyl and $R^8$ is 6-chloro;

1e1. $R^5$ is methyl;

1e2. $R^5$ is methoxy;

1e3. $R^5$ is chloro; or a pharmaceutically-acceptable salt thereof.

2. Those of the Formula:

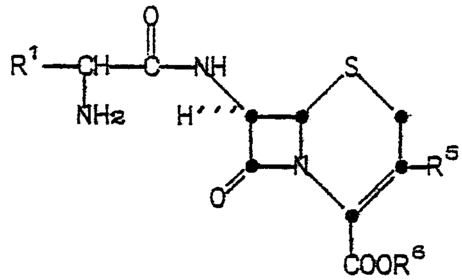

in which $R^1$ is

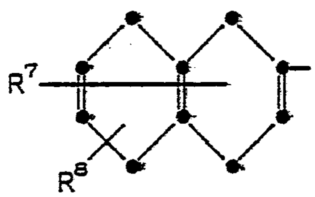

or

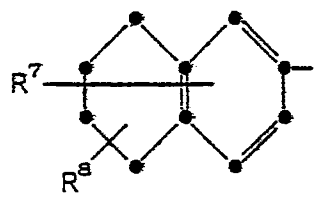

;

and $R^6$ is hydrogen or a salt cation.

a. $R^7$ and $R^8$ both are hydrogen;

2a1. $R^5$ is methyl;

2a2. $R^5$ is chloro;

2a3. $R^5$ is methoxy;

2a4. $R^5$ is hydrogen;

b. $R^7$ is hydrogen and $R^8$ is 7-hydroxy;

2b1. $R^5$ is methyl;

2b2. $R^5$ is fluoro;

2b3. $R^5$ is iodo;

2b4. $R^5$ is methoxy;

2b5. $R^5$ is hydrogen;

c. $R^7$ is 1-methoxy and $R^8$ is 8-tert-butyl;

2c1. $R^5$ is methyl;

2c2. $R^5$ is methoxy;

2c3. $R^5$ is chloro;

2c4. $R^5$ is methoxymethyl;

d. $R^7$ is 3-chloro and $R^8$ is 7-isopropoxy;

2d1. $R^5$ is hydrogen;

2d2. $R^5$ is methoxymethyl;

2d3. $R^5$ is methoxy;

2d4. $R^5$ is methyl;

2d5. $R^5$ is chloro;

or a pharmaceutically-acceptable salt thereof.

B. Those of the formula

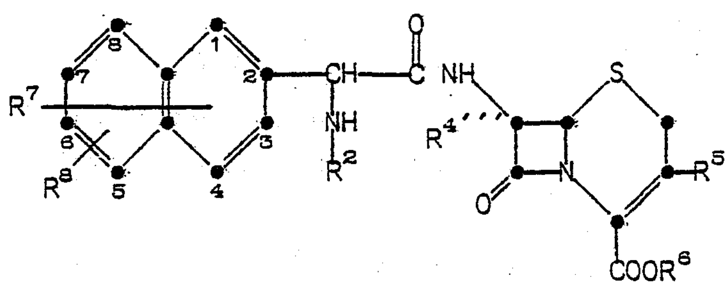

1. $R^4$ is hydrogen, $R^5$ is methyl;

a. $R^7$ and $R^8$ both are hydrogen; $R^2$ is tert-butoxycarbonyl;

1a1. $R^6$ is p-nitrobenzyl;

1a2. $R^6$ is 2,2,2-trichloroethyl;

1a3. $R^6$ is trimethylsilyl;

1a4. $R^6$ is phenacyl;

b. $R^7$ and $R^8$ both are hydrogen, $R^6$ is tert-butyl;

1b1. $R^2$ is tert-butoxycarbonyl;

1b2. $R^2$ is acetyl;

1b3. $R^2$ is p-nitrobenzyloxycarbonyl;

1b4. $R^2$ is chloroacetyl;

c. $R^7$ is hydrogen, $R^8$ is 7-methoxy, $R^2$ is 2,2,2-trichloroethoxycarbonyl;

1c1. $R^6$ is p-nitrobenzyl;

1c2. $R^6$ is sodium cation;

1c3. $R^6$ is methyl;

2. $R^4$ is methoxy, $R^5$ is chloro;

a. $R^7$ and $R^8$ both are hydrogen, $R^2$ is formyl;

2a1. $R^6$ is 2,2,2-trichloroethyl;

2a2. $R^6$ is hydrogen;

2a3. $R^6$ is sodium cation;

3. $R^4$ is methylthio, $R^7$ and $R^8$ both are hydrogen;

a. $R^5$ is methyl;

3a1. $R^2$ is tert-butoxycarbonyl;

3a2. $R^6$ is para-nitrobenzyl;

b. $R^5$ is bromo;

3b1. $R^2$ is tert-butoxycarbonyl;

3b2. $R^6$ is trimethylsilyl;

c. $R^5$ is methoxymethyl;

3c1. $R^2$ is hydrogen;

3c2. $R^2$ is trimethylsilyl;

3c3. $R^6$ is allyl;

4. $R^4$ is methylthio, $R^7$ is hydrogen and $R^8$ is 6-methoxy;

a. $R^5$ is methoxy;

4a1. $R^2$ is tert-butoxycarbonyl;

4a2. $R^6$ is methyl;

b. $R^5$ is chloro;

4b1. $R^2$ is hydrogen;

4b2. $R^2$ is phenacyl;

4b3. $R^6$ is tert-butyl;

C. Those of the Formula:

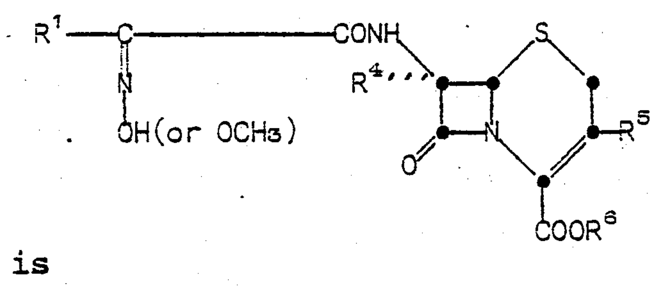

in which $R^1$ is

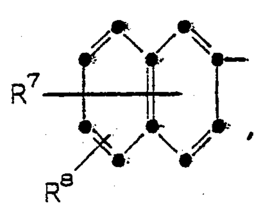 , 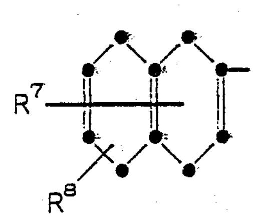 or 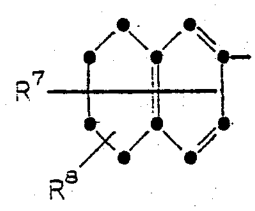

1. $R^7$ and $R^8$ both are hydrogen;
   a. $R^5$ is methyl;
      1a1. $R^6$ is <u>para</u>-nitrobenzyl;
      1a2. $R^6$ is <u>tert</u>-butyl;
      1a3. $R^6$ is hydrogen;
      1a4. $R^6$ is trimethylsilyl;

   b. $R^5$ is fluoro;
      1b1. $R^6$ is methyl;

   c. $R^5$ is methoxymethyl;

The following non-limiting examples are provided to further illustrate the invention.

<u>Preparation 1</u>

Preparation of 2-naphthylglycine (also named α-amino-α-(2-naphthyl)acetic acid)

A solution of 15.6 g (0.1 m) of 2-naphthaldehyde in 700 ml of 50% ethanol-water containing 14.7 g (0.3 m) of sodium cyanide and 38.4 g (0.4 m) of ammonium carbonate was heated at 50°C for twenty hours. The reaction mixture was cooled and concentrated to about 400 ml by evaporation under reduced pressure, and then the solution was made acidic to pH 2.0 by the addition of conc. hydrochloric acid. The solid precipitate that formed was collected by filtration, washed with dilute hydrochloric acid, and then dried to afford 22.1 g of 4-(2-naphthyl)-2,4-imidazolidindione.

A solution of 5.0 g (22 mM) of the 4-(2-naphthyl)-2,4-imidazolidindione in 100 ml of 16% (v/v) aqueous sodium hydroxide was heated at reflux for two and one-half hours. The reaction mixture was then filtered, cooled, and washed with ethylacetate. The aqueous solution was next diluted with 6N hydrochloric acid to pH 5.1 and filtered to provide 2-naphthylglycine. The reaction was repeated several times to produce larger quantities of the product.

A 10.0 g sample of 2-naphthylglycine was purified by dissolving it into 125 ml of methanol containing 3.9 ml of acetyl chloride. The reaction mixture was filtered and the filtrate was then diluted with 5 ml of aniline. The precipitated product was collected by filtration and dried to give 7.0 g of 2-naphthylglycine. m.p. 219-221°C.

## Preparation 2

### Resolution of 2-naphthylglycine

A mixture of D and L 2-naphthylglycine was reacted with optically pure α-aminoethylbenzene in the presence of N,N'-dicyclohexylcarbodiimide to provide N-(1-phenylethyl)-α-amino-α-(2-naphthyl)acetamide. Separation of the diastereomers by chromatography over silica gel afforded, following acid hydrolysis, D-2-naphthylglycine (OR -190° ± 3°) and L-2-naphthylglycine (OR = +190° ± 3°).

## Preparation 3

Preparation of 6-methoxynaphth-2-yl-glycine

2-Bromo-6-methoxynaphthalene was converted to the 2-lithio derivative by reaction with n-butyllithium. Diethyl oxalate was then reacted with the 2-lithio-6-methoxynaphthalene to afford ethyl α-keto-6-methoxy-naphth-2-ylacetate. The latter compound was reacted with hydroxylamine hydrochloride and sodium acetate to provide ethyl α-hydroxyimino-6-methoxynaphth-2-ylacetate. A solution of 17.55 g of the oxime in 600 ml of methanol containing 5.3 g of zinc metal dust and 135 ml of 50% (v/v) aqueous formic acid was stirred at 0°C for three hours. After filtering the reaction mixture, the solvent was removed by evaporation to give 10.3 g of ethyl α-amino-α-(6-methoxynaphth-2-yl)acetate. NMR ($CDCl_3$): δ 1.20 (t, 3H); δ 2.15 (s, 2H); δ 3.89 (s, 3H); δ 4.15 (m, 1H); δ 4.72 (s, 1H); δ 6.08-6.75 (m, 6H).

Hydrolysis of the ester thus formed by reaction with 1N sodium hydroxide afforded 6-methoxynaphth-2-ylglycine.

## Preparation 4

Preparation of N-*tert*-butoxycarbonyl-2-naphthylglycine

To a stirred solution of 10 g (50 mM) of 2-naphthylglycine (from Preparation 1) in 100 ml of 1N sodium hydroxide were added 50 ml of tetrahydrofuran

followed by 30 g (140 mM) of di-*tert*-butyl carbonate. The reaction mixture was stirred at 24°C for four hours. The product was isolated by first washing the reaction mixture three times with 50 ml portions of diethyl ether, and then the mixture was made acidic to pH 2.0 by the addition of conc. hydrochloric acid. The aqueous acid mixture was extracted several times with ethyl acetate, and the extracts were combined, washed with water, dried and the solvent was removed by evaporation under reduced pressure to provide 12.8 g (85% yield) of N-*tert*-butoxycarbonyl-2-naphthylglycine. NMR (DMSO-$d_6$): δ 2.5 (s, 9H); δ 6.85 (s, 1H); δ 7.28-7.9 (m, 7H).

By following the general procedures set out in Preparations 1-4 the following compounds were prepared:

N-*tert*-butoxycarbonyl-(6-methoxy-2-naphthyl)-glycine
NMR ($CDCl_3$): δ 1.2 and 1.4 (two broad singlets, 9H); δ 5.4 (broad singlet, 1H); δ 6.7 (broad singlet, 1H); δ 7.03-7.8 (m, 6H).

N-*tert*-butoxycarbonyl-(6-hydroxy-2-naphthyl)-glycine
NMR ($CDCl_3$): δ 1.2-1.4 (broad singlet, 9H); δ 5.3-5.9 (two broad singlets, 1H); δ 6.9-8.5 (m, 7H).

N-*tert*-butoxycarbonyl-(6-chloro-2-naphthyl)-glycine
NMR ($CDCl_3$): δ 1.15 (s, 9H); δ 5.3-5.7 (m, 1H); δ 7.3-8.3 (m, 8H).

## Preparation 5

α-Amino-α-(2-naphthyl)acetyl chloride hydrochloride

Hydrogen chloride was bubbled through a cold (0°C) solution of 5.0 g (25 mM) of 2-naphthylglycine in 150 ml of dichloromethane for twenty minutes. The reaction mixture was then stirred while 7.6 g (38 mM) of phosphorus pentachloride were added in one portion, and stirring was continued at 0-10°C for two hours. The solution was filtered, dried, and the solvent was removed by evaporation under reduced pressure to give 5.2 g (81% yield) of α-amino-α-(2-naphthyl)acetyl chloride hydrochloride. IR (mull) 1795 $cm^{-1}$

Analysis calculated for $C_{12}H_{11}Cl_2NO$

Theory: Cl, 27.68.

Found: Cl, 27.69.

## Preparation 6

Enzymatic resolution of D,L 2-naphthylglycine

By following the general procedure of U.S. Patent No. 3,386,888, 19.8 g of D,L-N-chloroacetyl-2-naphthylglycine was reacted with 4 g of N-acyl L-amino acid amidohydrolase in 1250 ml of 0.1M potassium hydrogen phosphate pH 7.0 buffer containing 5 x $10^{-4}$M cobalt chloride hexahydrate. The reaction mixture was shaken for two hours at 37°C. L-2-Naphthylglycine had precipitated and was removed by filtration. The filtrate was acidified to pH 2 by addition of 1N hydro-

chloric acid, and the mixture was extracted twice with 500 ml portions of ethyl acetate. The extracts were combined, dried, and the solvent was removed by evaporation under reduced pressure to give 9.065 g of D-N-chloroacetyl-2-naphthylglycine (91.5% yield).

Analysis calc. for $C_{14}H_{12}NO_3Cl$

Theory: C, 60.55; H, 4.36; N, 5.04.

Found: C, 60.62; H, 4.34; N, 4.76.

$[\alpha_D^{25}]$ -212.0°

The L-2-naphthylglycine that was collected by filtration was washed with pH 7.0 buffer, with water, and finally with hexane and air dried to give 6.82 g (95% yield) of L-2-naphthylglycine.

Analysis calc. for $C_{12}H_{11}NO_2$

Theory: C, 71.63; H, 5.55; N, 6.96.

Found: C, 69.85; H, 5.62; N, 6.51.

$[\alpha_D^{25}]$ +195.2°.

## Preparation 7

8-Nitro-2-napthoic acid

5-Nitro-2-napthoic acid

To a stirred solution of 400 ml concentrated nitric acid at 60°C were added portion-wise 18 g (0.11 M) of 2-napthoic acid. The reaction mixture was heated at 70°C for two hours, cooled, and added to 200 g of ice. The precipitate was collected by filtration and dried to give 18.8 g (77% yield) of a mixture of 5 and 8-nitro-2-napthoic acid. The mixture was converted to the ethyl ester by reaction with ethanol in the presence of

sulfuric acid. Five grams of the mixture of ethyl esters were crystallized from 20 ml of ethyl acetate to give 800 mg of ethyl 8-nitro-2-napthoate (m.p. 120°C) and 1.9 g of ethyl 5-nitro-2-napthoate.

Preparation 8

Ethyl 8-Amino-2-naphthylformate

A solution of 11.2 g of ethyl 8-nitro-2-napthoate (prepared as in Preparation 7) in 100 ml of ethanol was hydrogenated in the presence of 5% palladium on carbon. The reaction mixture was filtered and the solvent was removed from the filtrate to give ethyl 8-amino-2-naphthylformate.

Preparation 9

Ethyl 8-hydroxy-2-naphthylformate

To a cold (0°C) stirred solution of 9.5 g (44 mM) of ethyl 8-amino-2-naphthylformate in 150 ml of 6N sulfuric acid was added dropwise over ten minutes a solution of 3.1 g (45 mM) of sodium nitrite in 25 ml of water. The reaction mixture was stirred for fifty minutes and then added to a hot solution (90°C) of 90 ml of water in 10 ml of conc. sulfuric acid. The reaction mixture was stirred for ten minutes at 90°C, then cooled and extracted with dichloromethane. The extracts were combined, washed with brine, dried, and the solvent was removed to give, following purification by chromatography, 1.7 g of ethyl 8-hydroxy-2-naphthylformate. m.p. 136-137°C.

Following the same general procedure, 14.9 g of ethyl 8-amino-2-naphthylformate were reacted with <u>tert</u>-butyl nitrite and copper (II) chloride to give 11.5 g of ethyl 8-chloro-2-naphthylformate.

## Preparation 10

Ethyl 8-methoxy-2-naphthylformate

A solution of 216 mg of ethyl 8-hydroxy-2-naphthylformate (from Preparation 9) in 15 ml of acetone containing six drops of dimethyl sulfate and 150 mg of potassium carbonate was stirred at 25°C for twenty-four hours. The reaction solvent was removed and the product was dissolved in ethyl acetate, washed with 5% hydrochloric acid and with brine, dried and concentrated to give 200 mg of ethyl 8-methoxy-2-naphthylformate.

## Preparation 11

α-Methoxyimino-α-(8-chloro-2-naphthyl)acetic acid

A suspension of 9.2 g sodium hydride in 50 ml of N,N-dimethylformamide was added in one portion to a stirred solution of 5.6 g (24 mM) of ethyl 8-chloro-2-naphthylformate and 4.4 g (36 mM) of methyl methylthiomethyl sulfoxide in 10 ml of N,N-dimethylformamide. The reaction mixture was stirred for four hours at 25°C, and then concentrated to dryness. The product was dissolved in 250 ml of ethyl acetate and the solution was washed with 5% hydrochloric acid,

saturated sodium bicarbonate and brine. The solution was dried and the solvent was removed by evaporation to give 4.73 g (63% yield) of 1-oxo-1-(8-chloro-2-naphthyl)-2-methylthio-2-methylsulfinylethane. A solution of 3.12 g (10 mM) of the product in 150 ml of formic acid and 12 ml of acetic anhydride was stirred at 65°C for thirty minutes. To the reaction mixture were added 856 mg (4 mM) of sodium periodate and stirring was continued for an additional fifteen minutes. The reaction mixture was cooled and concentrated to dryness, and the product was dissolved in ethyl acetate, washed with sodium bicarbonate and brine, and the solvent was removed to give 1.2 g (46% yield) of methylthio α-oxo-α-(8-chloro-2-naphthyl)acetate.

A solution of 220 mg of the product from above in 15 ml of methanol and 15 ml of water containing 0.83 ml of 1N sodium hydroxide and 70 mg of methoxyamine hydrochloride was stirred for sixteen hours at 25°C. The reaction mixture was made acid to pH 2 by addition of 1N hydrochloric acid. The acid solution was extracted with ethyl acetate which was dried and concentrated to give 170 mg of α-methoxyimino-α-(8-chloro-2-naphthyl)acetic acid.

By following the same general procedure, the following compounds are prepared:

α-Methoxyimino-α-(8-nitro-2-naphthyl)acetic acid;

α-Methoxyimino-α-(8-amino-2-naphthyl)acetic acid;

α-Methoxyimino-α-(8-hydroxy-2-naphthyl)-acetic acid; and

α-Methoxyimino-α-(8-methoxy-2-naphthyl)-acetic acid.

## Preparation 12

Preparation of 7-amino-3-chloro-3-cephem-4-carboxylic acid

U.S. Patent No. 3,925,372 describes the synthesis of 7-phenylglycylamido-3-chloro-3-cephem-4-carboxylic acid, now generically known as cefaclor. Reaction of cefaclor with phosphorous pentachloride, methanol and water under known conditions for cleavage of cephalosporin side chains affords 7-amino-3-chloro-3-cephem-4-carboxylic acid.

Similarly, the following nuclei may be prepared for use in the synthesis of compounds of the present invention:

7-amino-7-methoxy-3-bromo-3-cephem-4-carboxylic acid;

7-amino-3-cephem-4-carboxylic acid;

7-amino-3-methoxymethyl-3-cephem-4-carboxylic acid;

7-amino-7-methylthio-3-methyl-3-cephem-4-carboxylic acid;

## Preparation 13

7-(D-2-naphthylglycylamido)-3-methyl-3-cephem-4-carboxylic acid N,N-dimethylformamide monohydrate

To a cold (-20 to -30°C) stirred suspension of 51.0 g of D-[N-(1-methoxycarbonyl)-2-propenyl]-2-naphthylglycine sodium salt, prepared by reacting methyl acetoacetate with D-2-naphthylglycine, in 500 ml of acetonitrile containing 250 ml of N,N-dimethylformamide were added sequentially 0.44 ml of methanesulfonic acid, 0.45 ml of N,N-dimethylbenzylamine and 12.35 ml of methyl chloroformate. The reaction mixture was stirred for two hours at -20 to -30°C following the addition. The reaction mixture was diluted by addition of a cold (0°C) solution of 34.24 g 7-amino-3-methyl-3-cephem-4-carboxylic acid in 230 ml of acetonitrile containing 59.4 ml of N-methyl monotrimethylsilyl trifluoroacetamide. The reaction mixture was stirred for two hours at -20°C to -30°C and then warmed to 0°C. The reaction mixture was acidified by addition of 160 ml of 1N hydrochloric acid, and the acidic mixture was stirred while 17.85 g of semicarbazide hydrochloride were added in one portion. The pH of the reaction mixture was adjusted to 3.0 and maintained by addition of triethylamine. After the mixture was warmed to 25°C it was filtered through Hyflo filter aid. The filtrate was diluted by addition of triethylamine to pH 6.2 and cooled to 0°C for forty-five minutes and then filtered. The filter cake was washed four times with 50 ml portions of acetonitrile and dried at 35°C for seven hours under reduced pressure to provide 66.8 g of 7-(D-2-naphthylglycylamido)-3-methyl-3-cephem-4-carboxylic acid N,N-dimethylformamide monohydrate. Yield 86%. Karl Fisher Assay: 4.69%.
NMR (TFA): signals at δ2.1(S,3H), 3.2(S,3H), 3.3(ABq,2H), 3.4(S,3H), 5.2(d,1H), 5.8(dd,1H), 5.8(S,1H), 7.3-8.2(m,7H), 8.3(S,1H).

## Example 1

7-(2-Naphthylglycylamido)-3-methyl-3-cephem-4-carboxylic acid trifluoroacetate salt

To a stirred suspension of 1.0 g (4.7 mM) of 7-amino-3-methyl-3-cephem-4-carboxylic acid (7-ADCA) in 25 ml of acetonitrile were added in one portion 3.7 ml (14.0 mM) of bis(trimethylsilyl)trifluoroacetamide. The reaction mixture was stirred at room temperature until all solids had dissolved, thus indicating complete formation of the trimethylsilyl ester of 7-ADCA.

In a separate flask a solution of 1.35 g (4.5 mM) of N-*tert*-butoxycarbonyl-2-naphthylglycine (from Preparation 4) in 20 ml of acetonitrile containing 1.1 g (4.5 mM) of N-ethoxycarbonyl-2-ethoxy-1,2-dihydroquinoline (EEDQ) was stirred at room temperature for fifteen minutes. This solution was then added in one portion to the cold (0°C) acetonitrile solution containing the trimethylsilyl ester of 7-ADCA from above. The reaction mixture was stirred for one hour at 0°C, and then warmed to room temperature. The solvent was removed by evaporation under reduced pressure to give an oil, and the oil was dissolved in ethyl acetate, washed two times with 1N hydrochloric acid,

dried, and the solvent was removed by evaporation to provide 7-(N-tert-butoxycarbonyl-2-naphthylglycylamido)-3-methyl-3-cephem-4-carboxylic acid as a foam.

The N-protected naphthylglycyl cephalosporin thus produced was dissolved in 5 ml of trifluoroacetic acid, and then the trifluoroacetic acid was removed by evaporation under reduced pressure to provide, following precipitation from diethyl ether, 5.7 g of 7-(2-naphthylglycylamido)-3-methyl-3-cephem-4-carboxylic acid trifluoroacetate salt.

## Example 2

7-(2-Naphthylglycylamido)-3-methyl-3-cephem-4-carboxylic acid tetrahydrate

A mixture of 5.7 g of the trifluoroacetic acid addition salt from Example 1 in 55 ml of 10% (v/v) water and acetonitrile was warmed to about 50°C and then filtered to remove the undissolved solids. The filtrate was then diluted with 1.8 molar ammonium hydroxide to pH 4.5. The precipitate that formed was collected by filtration and dried to give 3.15 g (72% yield) of 7-(2-naphthylglycylamido)-3-methyl-3-cephem-4-carboxylic acid tetrahydrate.

## Example 3

7-(D-2-Naphthylglycylamido)-3-methyl-3-cephem-4-carboxylic acid tetrahydrate

The procedure of Example 1 was repeated using 5.0 g of optically active D-N-tert-butoxycarbonyl-2-naphthylglycine and 5.6 g of 7-ADCA to provide, following removal of the N-protecting group, 6.8 g (80% yield) of D-7-(2-naphthylglycylamido)-3-methyl-3-cephem-4-carboxylic acid trifluoroacetic acid salt. The salt thus formed was dissolved in 90 ml of acetonitrile and 10 ml of water containing 5 ml of triethylamine. The reaction mixture was stirred at 25°C for twenty minutes and filtered. The filtrate was concentrated to dryness and the product was crystallized from water to give 2.9 g of 7-(D-2-naphthylglycylamido)-3-methyl-3-cephem-4-carboxylic acid tetrahydrate. m.p. 171-180°C (dec.)

Analysis calc. for $C_{20}H_{27}N_3O_8S$

Theory: C, 51.16; H, 5.80; N, 8.95; S, 6.93.

Found: C, 52.52; H, 5.47; N, 8.73; S, 6.83.

NMR (DMSO-$d_6$): δ 1.9 (s, 3H); δ 4.8 (s, 1H); δ 4.9 (dd, 1H); δ 5.6 (dd, 1H); δ 7.49-7.99 (m, 7H).

## Example 4

7-(D-2-Naphthylglycylamido)-3-methyl-3-cephem-4-carboxylic acid tetrahydrate

D-2-Naphthylglycine sodium salt was protected as an enamine by reaction with methyl acetoacetate. A suspension of 102 g (317.7 mM) of the protected D-2-naphthylglycine sodium salt in 1000 ml of acetonitrile and 500 ml of N,N-dimethylformamide was cooled to -30°C and stirred while 0.88 ml of methanesulfonic acid was added in one portion, followed by the addition of 0.90 ml of N,N-dimethylbenzylamine and 24.7 ml of methyl chloroformate. The reaction mixture was stirred at

-30°C for two hours, and was then diluted by dropwise addition of a solution of 68.5 g (302.8 mM) of 7-amino-3-methyl-3-cephem-4-carboxylic acid in 460 ml of acetonitrile containing 118.8 ml hexamethyldisilazane. The reaction mixture was stirred at -30°C for about two hours following complete addition and then warmed to 0°C. The reaction mixture was diluted by addition of 320 ml of 1N hydrochloric acid, followed by addition of 35.7 g of semicarbazide hydrochloride. Ammonium hydroxide was added to adjust and maintain the pH at 3.0 while the mixture was warmed to 22°C. The reaction mixture was further diluted by addition of 430 ml of water, and then decolorized by stirring for fifteen minutes with 10.0 g of charcoal. The reaction mixture was filtered through Hyflo filter aid and the filtrate was warmed to 40°C. The pH was adjusted to 4.0 by addition of 1N ammonium hydroxide, whereupon crystallization started. Crystallization continued for about thirty minutes, and then the pH was raised to 5.2 by addition of 1N ammonium hydroxide. The mixture was cooled to 20°C and stirred for one hour and filtered. The filter cake was washed twice with 50 ml portions of water and air dried to give 110.8 g of 7-(D-2-naphthylglycylamido)-3-methyl-3-cephem-4-carboxylic acid tetrahydrate.

Analysis calc. for $C_{20}H_{19}N_3O_4S \cdot 4H_2O$

Theory: C, 51.16; H, 5.80; N, 8.97; S, 6.83;

Found: C, 50.31; H, 5.62; N, 8.87; S, 6.89.

Karl Fisher water analysis:

Theory: 15.3

Found: 13.73%

NMR (TFA): δ 2.2 (s, 3H); 3.21 (q, 2H); 5.08 (d, 1H); 5.68 (m, 2H); 7.4-8.3 (m, 7H); 10.5 (s, 12H).
IR (KBr): 1766, 1751, 1696 $cm^{-1}$
UV ($CH_3OH$): $\lambda_{max}$ 227, ε, 78,000
$\lambda_{max}$ 265, ε, 12,000.
Titration (66% N,N-dimethylformamide/water)
pKa 5.5, 7.5, 11.2.

## Example 5

7-(D-2-Naphthylglycylamido)-3-methyl-3-cephem-4-carboxylic acid hydrochloride monohydrate

To a stirred solution of 200 ml of water containing 10 ml of 12N hydrochloric acid were added in one portion 20 g of 7-(D-2-naphthylglycylamido)-3-methyl-3-cephem-4-carboxylic acid N,N-dimethylformamide solvate (from Preparation 13). The reaction mixture was adjusted to pH 0.60 by addition of 12N hydrochloric acid and then stirred at 25°C for forty-five minutes. The precipitated solid was collected by filtration and washed one time with 50 ml of water. The crystalline material was dried at 35-40°C for sixteen hours to give 17.69 g (92%) of 7-(D-2-naphthylglycylamido)-3-methyl-3-cephem-4-carboxylic acid hydrochloride monohydrate.

Analysis calc. for $C_{20}H_{22}ClN_3O_5S$
Theory: C, 53.15; H 4.91; N, 9.30; S, 7.09; O, 17.70; Cl, 7.85
Found: C, 52.75; H, 4.85; N, 9.85; S, 7.03; O, 17.07; Cl, 7.60.

Karl Fisher Assay:
Theory: 4.29%
Found: 4.62%

Chloride Assay (by titration): Theory 7.85; Found 8.16 and 8.02. IR (KBr): 3040, 2940, 1768, 1707, 1533, 1232 $cm^{-1}$.
UV (EtOH): $\lambda_{max}$ 225, ε=100,000.
Titration (66% N,N-dimethylformamide-water v/v): 5.5, 7.3.

## Example 6

7-(D-2-Naphthylglycylamido)-3-methyl-3-cephem-4-carboxylic acid hydrochloride monohydrate

A solution of 5 g (10.6 mM) of 7-(D-2-naphthylglycylamido)-3-methyl-3-cephem-4-carboxylic acid tetrahydrate (from Example 3) in 50 ml of acetone was heated to 40°C and then acidified by addition of 1 ml of 12N hydrochloric acid. The mixture was stirred and diluted by addition of 5 ml of water and 6N ammonium hydroxide to pH 3.3. The pH was lowered to 0.20 by the dropwise addition of 12N hydrochloric acid, and then the reaction mixture was stirred for two hours at 25°C. The precipitated solid was collected by filtration, washed twice with 10 ml portions of water, and dried at 25°C under vacuum for two hours to afford 1.929 g (40.5% yield) of crystalline 7-(D-2-naphthylglycylamido)-3-methyl-3-cephem-4-carboxylic acid hydrochloride monohydrate.
Chloride analysis: Theory 7.85%; Found 7.96%.

Example 7

7-(D-2-Naphthylglycylamido)-3-methyl-3-cephem-4-carboxylic acid hydrochloride monohydrate

To a warm (40°C) stirred solution of 1 ml of 12N hydrochloric acid in 25 ml of water and 25 ml of acetone were added in one portion 5 g of 7-(D-2-naphthylglycylamido)-3-methyl-3-cephem-4-carboxylic acid tetrahydrate (from Example 3). Additional 12N hydrochloric acid was added to the reaction mixture to adjust the pH to 0.15, and then the mixture was cooled to 25°C and stirred for two hours. The precipitated solid was collected by filtration, washed with three 10 ml portions of water, and dried for four hours at 25°C under vacuum. The product was determined to be 3.596 g (75% yield) of 7-(D-2-naphthylglycylamido)-3-methyl-3-cephem-4-carboxylic acid hydrochloride monohydrate.

Karl Fisher Assay:
Theory: 4.29%
Found: 5.95%.

Chloride analysis: Theory 7.85%. Found 8.09%.

Example 8

7-(D-2-Naphthylglycylamido)-3-methyl-3-cephem-4-carboxylic acid hydrochloride monohydrate

A solution of 10 g (21.3 mM) of 7-(D-2-naphthylglycylamido)-3-methyl-3-cephem-4-carboxylic acid tetrahydrate in 100 ml of water containing 5 ml of 12N hydrochloric acid was stirred at 40°C while 12N hydrochloric acid was added dropwise to pH 0.6. The reaction mixture was cooled to 25°C and was stirred at that temperature for one hour. The crystalline product was collected by filtration, washed twice with 20 ml portions of fresh water, and dried for three hours at 25°C under reduced pressure. The dried product was identified as 8.16 g (84.5% yield) of crystalline 7-(D-2-naphthylglycylamido)-3-methyl-3-cephem-4-carboxylic acid hydrochloride monohydrate.

Karl Fisher analysis:

Theory: 4.29%

Found: 5.37%.

Chloride analysis: Theory 7.85%. Found 7.62%.

## Example 9

p-Nitrobenzyl 7-(N-tert-butoxycarbonyl-2-naphthylglycylamido)-3-chloro-3-cephem-4-carboxylate

A solution of 260 mg of EEDQ in 50 ml of acetonitrile containing 301 mg of N-tert-butoxycarbonyl-2-naphthylglycine was added in one portion to a stirred cold (0°C) solution of 682 mg of p-nitrobenzyl 7-amino-3-chloro-3-cephem-4-carboxylate in 50 ml of acetonitrile. The reaction mixture was stirred for ninety minutes at 0°C, and then was warmed to room temperature. The reaction solvent was removed by evaporation under reduced pressure to provide the product as an oil. The oil was dissolved in ethyl acetate and washed with dilute hydrochloric acid, pH 7.0 buffer, and finally with brine. After drying the

organic solution, the solvent was removed by evaporation to provide 598 mg (93% yield) of p-nitrobenzyl 7-(N-*tert*-butoxycarbonyl-2-naphthylglycylamido)-3-chloro-3-cephem-4-carboxylate.
NMR ($CDCl_3$): δ 1.21 (s, 9H); δ 3.1-3.8 (m, 2H); δ 4.9 (two doublets, 1H); δ 5.28 (s, 2H); δ 5.4-6.2 (m, 3H); δ 7.2-8.21 (m, 12H).

## Example 10

7-(2-Naphthylglycylamido)-3-chloro-3-cephem-4-carboxylic acid trifluoroacetate

To a solution of 598 mg of p-nitrobenzyl 7-(N-*tert*-butoxycarbonyl-2-naphthylglycylamido)-3-chloro-3-cephem-4-carboxylate (from Example 9) in 50 ml of tetrahydrofuran containing 10 ml of methanol and 5 ml of ethanol were added portionwise 1.20 g of 5% palladium on carbon. The reaction mixture was shaken for one hour in a Paar hydrogenation flask under an initial hydrogen pressure of 58 psi. The reaction mixture was then filtered and the solvent was removed from the filtrate by evaporation under reduced pressure to provide an oil. The oil was dissolved in pH 7.0 buffer, and the pH was adjusted to 7.75 by addition of dilute sodium hydroxide. The aqueous solution was washed with ethyl acetate and diethyl ether, and then acidified to pH 2.3 by addition of 1N hydrochloric acid. The acidic solution was extracted with ethyl acetate, which was then dried and the solvent was removed by evaporation to provide 360 mg (77% yield) of 7-(N-tert-butoxycarbonyl-2-naphthylglycylamido)-3-chloro-3-cephem-4-carboxylic acid.

The acid thus produced was dissolved in 6 ml of trifluoroacetic acid and the solution was stirred for five minutes at 25°C. The pH was then adjusted to 3.9 by addition of dilute ammonium hydroxide, and the solvents were removed by evaporation to provide an oil. The oil was subjected to gradient chromatography over reverse phase silica gel, eluting with 0-25% acetonitrile, 1% acetic acid and 99-74% water (v/v). The appropriate fractions were combined and the solvent was removed by lyophilization to afford 49 mg of L- and 49 mg of D-7-(2-naphthylglycylamido)-3-chloro-3-cephem-4-carboxylic acid.

NMR (TFA-$d_1$): δ 3.82 (q, 2H); δ 5.49 (d, 1H); δ 5.8-6.1 (m, 2H); δ 7.6-8.35 (m, 7H).

## Example 11

7-(2-Naphthylglycylamido)-3-methoxy-3-cephem-4-carboxylic acid trifluoroacetate

A solution of 1.05 g of D,L-N-tert-butoxycarbonyl 2-naphthylglycine in 15 ml of acetonitrile containing 1.06 g of EEDQ was stirred at 25°C for thirty minutes, and then was added in one portion to a stirred solution of 1.0 g of 7-amino-3-methoxy-3-cephem-4-carboxylic acid in 15 ml of acetonitrile containing 1 ml of bis(trimethylsilyl)trifluoroacetamide. The reaction mixture was stirred for two hours at 25°C and then concentrated to dryness by evaporation of the solvent

under reduced pressure to provide an oil. The oil was dissolved in 10 ml of trifluoroacetic acid and stored at 0°C for five minutes. Evaporation of the solvent and purification of the product by chromatography over silica gel afforded 89.5 mg of D,L 7-(2-naphthylglycylamido)-3-methoxy-3-cephem-4-carboxylic acid trifluoroacetate.

IR (KBr): 1762.10 $cm^{-1}$ (β-lactam);

NMR (TFA-$d_1$): δ 3.2-4.25 (m, 5H); δ 5.2-5.9 (m, 3H); δ 7.5-8.3 (m, 7H).

## Example 12

D-7-(6-chloronaphth-2-ylglycylamido)-3-methyl-3-cephem-4-carboxylic acid

2-Chloronaphthalene was acylated with ethyl chloro-oxalate to produce ethyl α-keto-α-(6-chloronaphth-2-yl)acetic acid. Reaction of the latter compound with hydroxylamine, followed by reduction and hydrolysis, provided 6-chloronaphth-2-ylglycine. This was converted to the N-*tert*-butoxycarbonyl protected derivative.

To a stirred cold (0°C) solution of 523 mg (1.5 mM) of *p*-nitrobenzyl 7-amino-3-methyl-3-cephem-4-carboxylate in 300 ml of acetonitrile was added a solution of 500 mg (1.5 mM) of N-*tert*-butoxycarbonyl-6-chloronaphth-2-ylglycine in 100 ml of acetonitrile containing 369 mg of EEDQ. The reaction mixture was stirred for one hour at 0°C., and then was warmed to room temperature and stirred for an additional forty-eight hours. The reaction solvent was next removed by

evaporation under reduced pressure to provide the product as an oil. The oil was dissolved in 100 ml of ethyl acetate and washed with 1N hydrochloric acid, aqueous sodium bicarbonate, water, and then dried. Removal of the solvent by evaporation afforded 770 mg of a white solid (77% yield) of p-nitrobenzyl 7-(N-tert-butoxycarbonyl-6-chloronaphth-2-ylglycylamido)-3-methyl-3-cephem-4-carboxylate.

NMR ($CDCl_3$): δ 1.40 (s, 9H); δ 2.12 (two singlets, 3H); δ 3.40 (q, 2H); δ 4.68 and 4.90 (two doublets, 1H); δ 5.30 (broad s, 3H); δ 5.6-6.0 (m, 1H); δ 7.2-8.3 (m, 8H).

Removal of the p-nitrobenzyl carboxy protecting group was accomplished by hydrogenation of 770 mg of the compound from above with 1.0 g of 5% palladium on carbon in 50 ml of methanol containing 20 ml of ethanol with an initial hydrogen pressure of 55 psi. The reaction was complete after fifty-five minutes, and the reaction mixture was filtered and the solvent was removed from the filtrate to give an oil. The oil was dissolved in 50 ml of ethyl acetate containing pH 7 buffer, and the aqueous layer was acidified to pH 2.3 with 1N hydrochloric acid. The product was extracted into ethyl acetate, which was then washed with water, dried and concentrated to dryness to afford 220 mg (36% yield) of 7-(N-tert-butoxycarbonyl-6-chloronaphth-2-ylglycylamido)-3-methyl-3-cephem-4-carboxylic acid.

NMR ($CDCl_3$): δ 1.46 (s, 9H); 2.15 (two singlets, 3H); δ 3.35 (m, 2H); δ 7.2-8.1 (m, 8H).

The product thus formed was dissolved in 5 ml of trifluoroacetic acid and the solution was stirred at room temperature for five minutes. Evaporation of the solvent and purification of the product by high pressure liquid chromatography provided D-7-(6-chloronaphth-2-ylglycylamido)-3-methyl-3-cephem-4-carboxylic acid.

## Example 13

D-7-(2-Naphthylglycylamido)-3-methoxymethyl-3-cephem-4-carboxylic acid

A solution of 570 mg of diphenylmethyl 7-amino-3-methoxymethyl-3-cephem-4-carboxylate tosylate in 30 ml of ethylacetate containing 10 ml of aqueous sodium bicarbonate was stirred for five minutes and then concentrated to dryness to give diphenylmethyl 7-amino-3-methoxymethyl-3-cephem-4-carboxylate as a white foam. The foam was dissolved in 20 ml of acetonitrile and added in one portion to a stirred solution of 301 mg of D-N-_tert_-butoxycarbonyl-2-naphthylglycine in 20 ml of acetonitrile containing 207 mg of $N,N^1$-dicylcohexylcarbodiimide and 135 mg of hydroxybenzotriazole. The reaction mixture was stirred at 25°C for four hours. The mixture was poured into 100 ml of ethyl acetate and the solution was washed once with 50 ml of aqueous sodium bicarbonate, once with 50 ml of 1N hydrochloric acid, once with water, dried, and the solvent was removed by evaporation under reduced pressure to give diphenylmethyl D-7-(N-_tert_-butoxycarbonyl-2-naphthylglycylamido)-3-methoxymethyl-3-cephem-4-carboxylate as a white foam. The foam was dissolved in 2 ml of triethylsilane and 5 ml of trifluoroacetic acid and the solution was stirred at 25°C for nine minutes. Removal of the solvent by evaporation under reduced pressure provided 260 mg of D-7-(2-naphthylglycylamido)-3-methoxymethyl-3-cephem-4-carboxylic acid trifluoroacetate salt.

The salt thus formed was dissolved in 5 ml of water and 5 ml of acetonitrile and the pH of the mixture was adjusted to 4.5 with 1N ammonium hydroxide. The solution was lyophilized to provide a white solid, which when purified by preparative reverse phase high pressure liquid chromatography afforded 20 mg of D-7-(2-naphthylglycylamido)-3-methoxymethyl-3-cephem-4-carboxylic acid.

NMR (DMSO-$d_6$): δ 3.13 (s, 3H); δ 3.28 (q, 2H); δ 4.12 (s, 2H); δ 4.95 (d, 1H); δ 5.65 (d, 1H); δ 7.41-4.23 (m, 7H).

## Example 14

p-Nitrobenzyl 7-[N-tert-butoxycarbonyl-(6-methoxy-2-naphthyl)glycylamido]-3-methyl-3-cephem-4-carboxylate

To a stirred solution of 662 mg (2 mM) of N-tert-butoxycarbonyl-(6-methoxy-2-naphthyl)glycine in 100 ml of acetonitrile containing 500 mg (2 mM) of N-ethoxycarbonyl-2-ethoxy-1,2-dihydroquinoline were added in one portion 770 mg (2.2 mM) of p-nitrobenzyl 7-amino-3-methyl-3-cephem-4-carboxylate. The reaction mixture was stirred at 25°C for sixteen hours and then

concentrated to dryness to provide an oil. The oil was dissolved in 50 ml of ethyl acetate and the solution was washed with 25 ml of 1N hydrochloric acid, 25 ml of aqueous sodium bicarbonate and water. The solution was dried and concentrated to dryness to provide 1.3 g of p-nitrobenzyl 7-[N-tert-butoxycarbonyl-(6-methoxy-2-naphthyl)glycylamido]-3-methyl-3-cephem-4-carboxylate. NMR ($CDCl_3$): δ 1.39 (s, 9H); δ 2.08 and 2.15 (two singlets, 3H); δ 3.34 (q, 2H); δ 3.90 (s, 3H); δ 4.9 (m, 1H); δ 5.29 (s, 2H); δ 5.31 (s, 1H); δ 5.68 (m, 1H); δ 7.08-8.25 (m, 12).

## Example 15

D-7-(6-Methoxy-2-naphthyl)glycylamido-3-methyl-3-cephem-4-carboxylic acid

To a stirred suspension of 1.4 g of 5% palladium on carbon in 50 ml of ethanol were added in one portion 1.3 g of p-nitrobenzyl 7-[N-tert-butoxycarbonyl-(6-methoxy-2-naphthyl)glycylamido]-3-methyl-3-cephem-4-carboxylate. The reaction mixture was stirred for three hours at 25°C under 55 psi hydrogen. The reaction mixture was then filtered and the filter cake was washed with fresh ethanol. The filtrate was concentrated to dryness by evaporation under reduced pressure to provide 1.1 g of 7-[N-tert-butoxycarbonyl-(6-methoxy-2-naphthyl)glycylamido]-3-methyl-3-cephem-4-carboxylic acid.

The acid thus formed was dissolved in 5 ml of trifluoroacetic acid and the solution was stirred at 25°C for five minutes. The reaction mixture was added

to 20 ml of water and the aqueous solution was lyophilized for twelve hours to give D,L-7-(6-methoxy-2-naphthylglycylamido)-3-methyl-3-cephem-4-carboxylic acid trifluoroacetate. The salt thus formed was redissolved in fresh water and purified by high performance liquid chromatography to afford D-7-(6-methoxy-2-naphthylglycylamido)-3-methyl-3-cephem-4-carboxylic acid.

NMR (TFA-$d_1$): δ 2.42 (s, 3H); δ 3.50 (q, 2H); δ 4.30 s, 3H); δ 5.39 (d, 1H); δ 5.8-6.1 (m, 2H); δ 7.42-8.30 (m, 6H).

## Example 16

Following the general procedure of Examples 14 and 15 p-nitrobenzyl 7-amino-3-methyl-3-cephem-4-carboxylate was reacted with D,L-N-*tert*-butoxycarbonyl-(6-hydroxy-2-naphthyl)glycine in the presence of EEDQ to provide p-nitrobenzyl D,L-7-[N-*tert*-butoxycarbonyl-(6-hydroxy-2-naphthyl)glycylamido]-3-methyl-3-cephem-4-carboxylate.

NMR ($CDCl_3$): δ 1.41 (s, 9H); δ 2.02 and 2.10 (two singlets, 3H); δ 2.9-3.5 (m, 2H); δ 4.9 (m, 1H); δ 5.23 (s, 2H); δ 5.35 (m, 1H); δ 5.6-6.0 (m, 2H); δ 6.72-8.21 (m, 12H).

The compound thus prepared was reacted with hydrogen and 5% palladium on carbon to give D,L-7-[N-*tert*-butoxycarbonyl-(6-hydroxy-2-naphthyl)glycylamido]-3-methyl-3-cephem-4-carboxylic acid.

NMR ($CDCl_3$): δ 1.15 (s, 9H); δ 1.82 and 1.89 (two singlets, 3H); δ 4.65 (two doublets, 1H); δ 5.23 (m, 1H); δ 6.0 (m, 1H); δ 6.7-7.9 (m, 7H); δ 8.5 (m, 2H).

Reaction of the compound from above with trifluoroacetic acid afforded D,L 7-(6-hydroxy-2-naphthylglycylamido)-3-methyl-3-cephem-4-carboxylic acid. The isomers were separated by high performance liquid chromatography to give D-7-(6-hydroxy-2-naphthylglycylamido)-3-methyl-3-cephem-4-carboxylic acid.

Example 17

D-7-(2-Naphthylglycylamido)-3-cephem-4-carboxylic acid trifluoroacetate

To a stirred suspension of 2.0 g (10 mM) of 7-amino-3-cephem-4-carboxylic acid in 15 ml of acetonitrile were added in one portion 8 ml (30 mM) of bis-(trimethylsilyl)trifluoroacetamide. The mixture was stirred at 25°C for thirty minutes, and then was cooled to 0°C and added in one portion to a stirred solution of 2.0 g (6.6 mM) of D,L-N-*tert*-butoxycarbonyl-2-naphthylglycine in 15 ml of acetonitrile containing 1.73 g (7.0 mM) of EEDQ. The reaction mixture was stirred for one hour at 25°C and then was concentrated to dryness to provide an oil. The oil was dissolved in 100 ml of ethyl acetate and washed four times with 25 ml portions of 1N hydrochloric acid, twice with brine, and dried. The solvent was removed by evaporation under reduced pressure to provide a white foam. The foam was dissolved in 25 ml of trifluoroacetic acid and the solution was sonicated for five minutes at 25°C. The

reaction mxiture was concentrated to dryness and triturated with diethyl ether to afford 1.8 g (55% yield) of D,L-7-(2-naphthylglycylamido)-3-cephem-4-carboxylic acid trifluoroacetate. The product thus produced was chromatographed over reverse phase $C_{18}$ silica gel, eluting with 8 liters of a solution of 1% acetic acid plus a gradient of 95% water -5% acetonitrile to 85% water -15% acetonitrile (v/v). The appropriate fractions were combined and the solvent was removed by lyophilization to afford 157 mg of D-7-(2-naphthylglycylamido)-3-cephem-4-carboxylic acid.
IR (KBr): 1771.75 $cm^{-1}$ (β-lactam);
NMR (TFA-$d_1$): δ 3.55 (q, 2H); δ 4.08 (s, 1H); δ 5.6-6.0 (m, 2H); δ 7.5-8.1 (m, 7H).

## Example 18

7-[α-Methoxyimino-α-(8-chloro-2-naphthyl)-acetamido]-3-methyl-3-cephem-4-carboxylic acid

Four hundred twenty milligrams of α-methoxyimino-α-(8-chloro-2-naphthyl)acetic acid were converted to the acid chloride by reaction with excess chlorine and 500 mg of triphenyl phosphite in 20 ml of dichloromethane. The reaction mixture was added in one portion to a stirred solution of 350 mg of 7-amino-3-methyl-3-cephem-4-carboxylic acid in 5 ml of dichloromethane containing 2 ml of bis(trimethylsilyl)trifluoroacetamide. The reaction mixture was stirred at 25°C for six hours and then diluted by addition of 20 ml of methanol. The solvent was removed by evaporation under reduced pressure to provide 7-[α-methoxyimino-α-(8-chloro-2-

naphthyl)acetamido]-3-methyl-3-cephem-4-carboxylic acid. The product thus formed is dissolved in formic acid containing zinc metal dust to give, following isolation and purification, 7-(8-chloro-2-naphthyl)glycylamido-3-methyl-3-cephem-4-carboxylic acid.

The following compounds are similarly prepared:

7-(8-nitro-2-naphthyl)glycylamido-3-chloro-3-cephem-4-carboxylic acid;

7-(8-hydroxy-2-naphthyl)glycylamido-3-methoxymethyl-3-cephem-4-carboxylic acid;

7-(8-amino-2-naphthyl)glycylamido-3-methyl-3-cephem-4-carboxylic acid; and

7-(8-methoxy-2-naphthyl)glycylamido-3-methyl-3-cephem-4-carboxylic acid.

The naphthylglycyl cephalosporins provided by this invention are valuable antibiotic substances, or intermediates therefor. The compounds, while active against a broad spectrum of gram-positive and gram-negative bacilli, are particularly effective against a wide variety of gram-positive bacilli. The antibiotic compounds are especially useful for treating infections in animals caused by gram-positive microorganisms. The compounds are particularly effective in the treatment of upper respiratory infections and similar diseases caused by H. influenza, S. aureus, S. pyogenes, and the like. The compounds are also effective in the treatment of diseases caused by anaerobic cocci such as Peptostreptococcus anaerobius, Peptostrept. intermedius, Peptostrept. productus, Peptococcus saccharolyticus, P.

prevotii, P. avaerobius, Propionibacterium acnes, Fusobacterium necrophorum, and the like.

A typical and preferred compound provided by this invention is 7-(2-naphthylglycylamido)-3-methyl-3-cephem-4-carboxylic acid, the compound of Example 3. The antibacterial activity of this compound and several others of the invention has been determined in standard in vitro agar dilution assays against a variety of gram positive microorganisms. The following Tables present typical minimum inhibitory concentrations (MIC's) in µg/ml for the compounds when evaluated against the indicated microorganisms. MIC's for several known compounds are also presented for comparison.

Table I

Agar Dilution MIC (μg/ml)

| Organism | Strain | Ampi-cillin | Cepha-lexin | Compound of Ex. 3 | Ex. 10A[a] | Ex. 10B[b] | Ex. 11 | Ex. 12 | Ex. 15 | Ex. 16 | Ex. 17 |
|---|---|---|---|---|---|---|---|---|---|---|---|
| Staph. aureus | X1.1 | 0.25 | 4 | 0.5 | 0.25 | 8 | 1 | 0.25 | 0.25 | 0.5 | 4 |
| | V41 | 32 | 128 | 8 | 16 | >128 | 32 | 8 | 16 | 8 | 64 |
| | X400 | 128 | 128 | 64 | 128 | >128 | 128 | 64 | 64 | 32 | 128 |
| | S13E | 64 | 128 | 8 | 16 | >128 | 32 | 8 | 16 | 8 | 64 |
| Staph. epi | EPI1 | 8 | 32 | 1 | 2 | 32 | 8 | 4 | 4 | 4 | 32 |
| | 222 | 0.25 | 8 | 0.5 | 0.25 | 4 | 2 | 0.5 | 1 | 1 | 4 |
| Strep. A | C203 | 0.03 | 0.5 | 0.5 | 0.125 | 4 | 0.125 | 0.06 | 0.125 | 0.125 | 0.5 |
| Strep. PN | PARK | 0.03 | 2 | 0.5 | 0.125 | 4 | >128 | 0.25 | 0.125 | 0.25 | 1 |
| H. influ. | BRUN | 0.5 | 8 | 8 | 4 | >128 | >128 | 32 | 16 | 8 | 64 |
| | 251 | 16 | 8 | 2 | 2 | 16 | 32 | 2 | 4 | 4 | 16 |
| Klebsiella | X26 | 16 | 4 | 2 | 2 | 64 | 8 | 8 | 4 | 4 | 16 |

[a]D- form

[b]L- form

<u>Table II</u>

Expanded Spectrum MIC (μg/ml)

| Organism | Strain | Compound of Ex. 3 | Ex. 12 | Ex. 13 | Ex. 15 | Ex. 16 |
|---|---|---|---|---|---|---|
| Staph. epi | EPI1 | 8 | 8 | 4 | 4 | 4 |
| | 270 | 4 | 4 | 2 | 4 | 4 |
| | 219 | 0.5 | 0 | 0.5 | 5 | 0.5 |
| | 269 | 2 | 4 | 2 | 2 | 2 |
| | 285 | 2 | 2 | 1 | 2 | 2 |
| | 286 | 1 | 1 | 0.5 | 0.5 | 1 |
| Staph. aureus | S224 | 0.5 | 0.5 | 0.5 | 0.5 | 0.5 |
| | S225 | 0.5 | 0.5 | 0.5 | 0.5 | 0.5 |
| | S226 | 1 | 1 | 1 | 1 | 1 |
| | S227 | 1 | 0.5 | 0.5 | 1 | 0.5 |
| | S228 | 0.5 | 0.5 | 0.5 | 0.5 | 0.5 |
| | S229 | 1 | 1 | 1 | 1 | 1 |
| | S230 | 0.5 | 0.5 | 0.5 | 0.5 | 0.5 |
| | S231 | 0.5 | 0.25 | 0.5 | 0.25 | 0.5 |
| | S234 | 1 | 1 | 1 | 1 | 1 |
| | S237 | 1 | 0.5 | 0.5 | 1 | 1 |
| | S238 | 1 | 1 | 1 | 1 | 1 |
| | S239 | 1 | 1 | 1 | 1 | 1 |

Table II cont'd.

Expanded Spectrum MIC (μg/ml)

| Organism | Strain | Compound of Ex. 3 | Ex. 12 | Ex. 13 | Ex. 15 | Ex. 16 |
|---|---|---|---|---|---|---|
| H. influ. | C.L. | 8 | 32 | 8 | 8 | 4 |
| | 76 | 2 | 16 | 8 | 4 | 2 |
| | HESS | 8 | 32 | 8 | 8 | 4 |
| | STEL | 8 | >64 | >64 | 16 | >64 |
| | 312 | 8 | 8 | 8 | 4 | 4 |
| | R465 | 8 | 32 | 16 | 16 | 16 |
| | 1930 | 8 | 16 | 16 | 8 | 4 |
| | 4842 | 4 | 8 | 8 | 4 | 4 |
| | 1683 | 2 | 2 | 4 | 2 | 2 |
| | M366 | 8 | >64 | >64 | 16 | >64 |
| | M370 | 4 | 8 | 4 | 4 | 4 |
| | M371 | 4 | 8 | 4 | 4 | 2 |
| | 105 | 4 | 8 | 8 | 4 | 4 |
| | 158 | 4 | 4 | 8 | 4 | 2 |
| | 164 | 4 | 4 | 8 | 4 | 2 |
| | 171 | 4 | 8 | 8 | 4 | 4 |
| | 169 | 4 | 8 | 8 | 4 | 4 |

Table III

Expanded Spectrum MIC (μg/ml)

| Organism | Strain | Cephalexin | Cefaclor | Compound of Example 3 |
|---|---|---|---|---|
| S. Aureus | 8725 | 2 | 2 | 0.25 |
| | 8787 | 1 | 1 | 0.25 |
| | 9050 | 2 | 2 | 0.5 |
| | 9290 | 4 | 8 | 1.0 |
| | 9767 | 4 | 2 | 1.0 |
| | 8438 | 1 | 1 | 0.25 |
| | 9136 | 2 | 2 | 0.25 |
| B. Fragilis | 10817 | 16 | 64 | 2 |
| | 180-821 | 16 | 64 | 2 |
| | 10695 | 64 | 32 | 4 |
| | 107268 | 16 | 16 | 8 |
| | 107266 | 32 | 16 | 16 |
| | 10750 | 64 | >64 | 16 |
| | 10774 | >64 | >64 | 16 |
| | 10837 | 16 | >64 | 16 |
| | 10683 | >64 | 16 | 32 |
| | 10668 | >64 | 64 | 32 |
| H. Influenza | 101 | 8 | 2 | 4 |
| | 102 | 16 | 16 | 32 |
| | 103 | 16 | 2 | 4 |
| | 104 | 16 | 1 | 1 |
| | 105 | 4 | 1 | 2 |
| | 106 | 8 | 2 | 1 |
| | 107 | 8 | 2 | 4 |
| | 108 | 8 | 1 | 4 |
| | 109 | 16 | 2 | 4 |
| | 110 | 8 | 1 | 4 |
| | 111 | 16 | 1 | 1 |

Table IV

Susceptibility of Anaerobic Cocci Isolates by the Agar-Dilution Method-24 hour MIC's (μg/ml)

| Anaerobic Coccus | | Cefoxitin | Compound of Example 3 |
|---|---|---|---|
| Peptococcus Asaccharolyticus | 1344 | 0.25 | ≤0.125 |
| Peptococcus Constellatus | 1468 | 8 | 0.25 |
| P. magnus | 1401 | 2 | 0.25 |
| P. magnus | 1477 | 0.5 | 0.5 |
| P. prevoti | 1293 | 1.0 | 0.5 |
| Peptostreptococcus anaerobius | 8 | 0.25 | ≤0.125 |
| " | 52 | 2 | 0.25 |
| " | 1477 | 1 | ≤0.125 |
| Peptostreptococcus intermedius | 1264 | 1.0 | 1.0 |
| " | 1524 | 4.0 | 1.0 |
| " | 1624 | 4.0 | 1.0 |
| Bacteroides fragilis | 3625 | 16 | 16 |
| | 7371A | 8 | 16 |
| | 200 | 8 | 32 |
| | 206 | 16 | 16 |
| | 19671 | 16 | >64 |
| | 19681 | 32 | 16 |

Table IV cont'd.

| Anaerobic Coccus | | Cefoxitin | Compound of Example 3 |
|---|---|---|---|
| Propionibacterum acnes | 44 | 2 | 4 |
| | 79 | 8 | 8 |
| | 101 | 4 | 8 |
| | 104 | 4 | 8 |
| | 105 | 2 | 4 |
| | 5191 | ≤0.06 | 0.25 |
| | 5227 | 2 | 0.25 |
| | 5228 | 4 | 8 |
| | 5229 | 2 | 8 |
| | 5246 | 4 | 8 |

The data in the above Tables clearly demonstrate the potent antibacterial activity possessed by the compounds provided by this invention.

In addition to possessing potent antibacterial activity against a wide variety of microorganisms, particularly gram positive organisms and anaerobes, the compounds of this invention also have demonstrated very favorable pharmacokinetics in animals. For example, when 7-(2-naphthylglycylamido)-3-methyl-3-cephem-4-carboxylic acid was administered to rats at an intravenous dose of 20 mg/kg, the plasma concentration after one hour was 18.6 μg/ml; after four hours, 14.1 μg/ml; and after twenty-four hours, the plasma level was still measured as 1.96 μg/ml. The compound is efficiently orally absorbed in rodents and exhibits higher and longer blood levels than cefaclor and cephalexin.

The compounds of the invention also have good stability to β-lactamases. Table IV shows the results of comparative studies of several cephalosporins (lower numbers mean greater stability to the indicated β-lactamase).

Table V

Stability to β-lactamases

| | Organism | | | | |
|---|---|---|---|---|---|
| | 265A | PS185 | TEM | 1082E | 1313G |
| cefaclor | 138 | 71 | 23 | 65 | 4605 |
| cephalexin | 72 | 37 | 1 | 6 | 254 |
| 7-(2-naphthylglycylamido)3-methyl-3-cephem-4-carboxylic acid | 50 | 25 | 3 | 26 | 256 |

The favorable pharmacokinetics of the compounds provided by this invention, coupled with their excellent oral antibacterial activity and stability toward β-lactamases, make them particularly attractive agents for the treatment of a number of diseases of bacterial origin. The compounds are especially well suited for the treatment of out-patients, and especially for subjects suffering from mild upper respiratory infections caused by gram positive microorganisms.

A means for treatment of animals suffering from bacterial diseases, or suspected of developing a bacterial infection, also is provided by this invention. The antibacterial method of treatment provided may be practiced by administering to an animal in need of treatment an antibacterially-effective amount of a

naphthylglycyl cephalosporin antibiotic. The method can be practiced therapeutically or prophylactically. The amount of active antibiotic to be administered will vary depending upon the particular compound selected for use, the severity of the disease being treated or guarded against, the individual undergoing treatment, and related factors commonly encountered with such treatments. Normally, however, the compounds will be administered at a dose of about 0.5 to about 50 mg/kg of animal body weight, and more preferably at a rate of about 1 to about 10 mg/kg. Such amounts may be administered once or twice each day, or more often as needed to treat the particular disease or subject undergoing treatment. A typical daily dose for an average adult human will be about 200 to about 500 mg per day.

The antibiotic compounds provided by this invention are active by both the oral and parenteral routes of administration, and accordingly can be formulated for any such desired route of administration. Such formulations constitute yet another embodiment of this invention. The formulations of this invention will comprise from about 0.1 to about 95 percent by weight of an active naphthylglycyl cephalosporin antibiotic of the invention, admixed with a pharmaceutically acceptable carrier, diluent or excipient therefor. Typical formulations will contain from about 10 to about 60 percent by weight of active ingredient, and more preferably about 20 to about 50 percent.

For convenient oral administration, the compounds can be admixed with any of a number of diluents, excipients and carriers commonly employed in oral

formulations, and molded into tablets, pills, troches, or encapsulated into gelatin capsules. Typical carriers, diluents and excipients commonly employed include potato starch, corn starch, sucrose, dextrose, microcrystalline cellulose, dicalcium phosphate, alginic acid, acacia; lubricants such as magnesium stearate; binders such as gum tragacanth or gelatin; and flavoring agents such as peppermint oil, cherry or strawberry flavoring, oil of wintergreen, and the like. The compounds also can be formulated as syrups or elixirs employing common diluents such as a fatty oil, methyl or propylparabens, suitable dyes and flavoring agents. The compounds can also be formulated in the form of a buccal seal, lozenge or other suitable device for sustained controlled delivery of the active ingredient over a prolonged period.

The antibiotics of the invention also can be formulated for parenteral administration, for example via the intravenous, intramuscular or subcutaneous routes, as well as the transdermal route. Such compositions normally will contain from about 0.1 to about 20.0 percent by weight of active ingredient. Typical excipients, diluents and carriers for parenteral formulations may include, for example, isotonic saline, dilute aqueous dextrose, the polyhydric aliphatic alcohols or mixtures thereof, for instance glycerin, propylene glycol, or polyethylene glycol. Parenteral solutions may also contain preservatives such as phenethyl-alcohol, methyl and propyl parabens, or thimerosal. If needed, about 0.05 to about 0.20 percent by weight of an antioxidant such as sodium metabisulfite or sodium bisulfite also can be employed. For intravenous use,

preferred formulations will employ an initial concentration down to about 0.05 to about 0.25 mg/ml of active ingredient, and for intramuscular injection, a preferred concentration of active ingredient is about 0.25 to about 0.50 mg/ml.

The following examples illustrate typical formulations which may be used.

## Example 19

### Formulation of Oral Suspension

| Ingredient | Amount |
|---|---|
| Sodium D-7-(2-Naphthylglycylamido)-3-chloro-3-cephem-4-carboxylate | 500 mg |
| Sorbitol solution (70% N.F.) | 40 ml |
| Sodium benzoate | 150 mg |
| Saccharin | 10 mg |
| Cherry flavor | 50 mg |
| Distilled water q s ad | 100 ml |

The sorbitol solution is added to 40 ml of distilled water and the naphthylglycyl cephalosporin is suspended therein. The saccharin, sodium benzoate, and flavoring are added and dissolved. The volume is adjusted to 100 ml with distilled water. Each ml of syrup contains 5 mg of the antibiotic naphthylglycyl cephalosporin. This oral formulation is suited ideally for pediatric use.

Example 20

Preparation of 250 mg capsule

| Ingredient | Amount |
| --- | --- |
| 7-(6-Chloronaphth-2-ylglycylamido)-3-methyl-3-cephem-4-carboxylic acid | 250 mg |
| Lactose | 150 mg |
| Corn starch | 100 mg |
| | 500 mg |

The ingredients are blended to uniformity and encapsulated into gelatin capsules. Such capsules may be administered orally, for example, at the rate of about one or two each day.

Example 21

Preparation of Parenteral Solution

In a solution of 700 ml of propylene glycol and 200 ml of distilled water for injection is dissolved 20.0 grams of D-7-(2-naphthylglycylamido)-3-methyl-3-cephem-4-carboxylic acid, hydrochloride. The pH of the solution is adjusted to 5.5 with hydrochloric acid, and the volume is made up to 1000 ml with distilled water. The formulation is sterilized, filled into 5.0 ml ampoules each containing 2.0 ml (representing 40 mg of active ingredient) and sealed under nitrogen.

## CLAIMS

1. A compound of Formula (I):

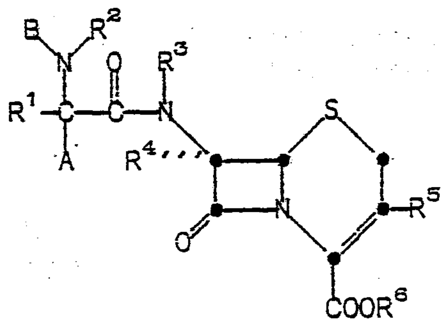

(I)

in which:

$R^1$ is

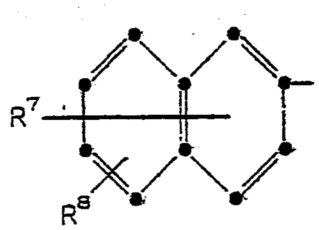

,

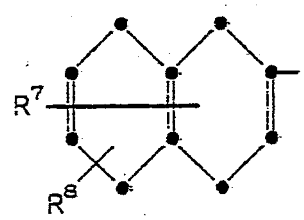

, or

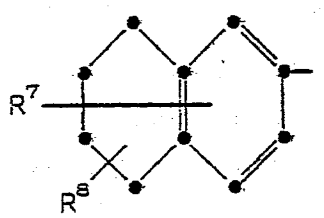

in which $R^7$ and $R^8$, independently, are hydrogen, halo, hydroxy, $C_1$-$C_4$ alkyl, $C_1$-$C_4$ alkoxy, nitro, amino, $C_1$-$C_4$ alkanoylamino, $C_1$-$C_4$ alkylsulfonylamino, or when $R^7$ and $R^8$ are taken together they form methylenedioxy;

A and B, both, are hydrogen, or taken together complete a double bond;

$R^2$ is hydrogen, an amino protecting group, hydroxy, or methoxy, and $R^3$ is hydrogen, or $R^2$ and $R^3$ taken together are

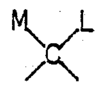 ,

where M and L, independently, are $C_1$-$C_4$ alkyl;

$R^4$ is hydrogen, methoxy or methylthio;

$R^5$ is hydrogen, methoxy, methyl, halo, or methoxymethyl;

$R^6$ is hydrogen, or a carboxy protecting group; provided that $R^2$ is hydroxy or methoxy only when A and B complete a double bond, and that A and B both are hydrogen when $R^3$ is other than hydrogen; or a pharmaceutically-acceptable salt thereof.

2. A compound of Formula (I), as claimed in claim 1, in which $R^2$ and $R^3$, when taken together, form

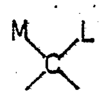 , ;

or a pharmaceutically-acceptable salt thereof.

3. A compound of Formula (I), as claimed in claim 1, in which A and B, when taken together, complete a double bond and $R^2$ is methoxy; or a pharmaceutically-acceptable salt thereof.

4. A compound of Formula (I), as claimed in any one of claims 1 to 3, in which $R^1$ is

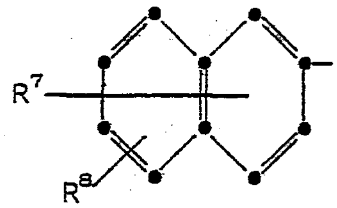

;

or a pharmaceutically-acceptable salt thereof.

5. A compound of Formula (I), as claimed in any one of claims 1 to 3 in which $R^1$ is

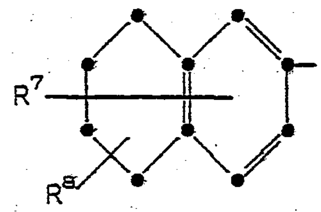

;

or a pharmaceutically-acceptable salt thereof.

6. A compound of Formula (I), as claimed in any one of claims 1 to 3 in which $R^1$ is

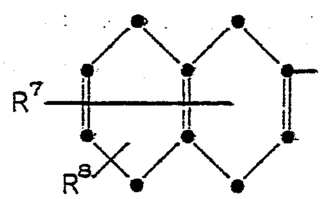

;

or a pharmaceutically-acceptable salt thereof.

7. A compound of Formula (I), as claimed in any one of claims 4 to 6, in which $R^7$ or $R^8$, independently, is hydrogen, halo, hydroxy, $C_1$-$C_4$ alkoxy, nitro or amino; or a pharmaceutically-acceptable salt thereof.

8. A compound of Formula (I), as claimed in claim 7, in which $R^7$ or $R^8$, independently, is hydrogen, halo, methoxy or hydroxy; or a pharmaceutically-acceptable salt thereof.

9. A compound of Formula (I), as claimed in claim 7 or 8, in which $R^7$ and $R^8$, both, are hydrogen; or a pharmaceutically-acceptable salt thereof.

10. A compound of Formula (I), as claimed in any one of claims 1 to 9, in which $R^5$ is methyl, chloro, hydrogen, methoxymethyl, or methoxy; or a pharmaceutically-acceptable salt thereof.

11. A compound of Formula (I), as claimed in claim 10, in which $R^5$ is methyl or chloro; or a pharmaceutically-acceptable salt thereof.

12. A compound of Formula (I), as claimed in any one of claims 1 to 11, in which $R^4$ is hydrogen; or a pharmaceutically-acceptable salt thereof.

13. A compound of Formula (I), as claimed in any one of claims 1 to 12, in which $R^6$ is hydrogen; or a pharmaceutically-acceptable salt thereof.

14. 7-[D-(2-naphthylglycylamido)]-3-methyl-3-cephem-4-carboxylic acid; or a pharmaceutically-acceptable salt thereof.

15. 7-[D-(2-naphthylglycylamido)]-3-methyl-3-cephem-4-carboxylic acid tetrahydrate.

16. 7-[D-(2-naphthylglycylamido)]-3-methyl-3-cephem-4-carboxylic acid hydrochloride monohydrate.

17. 7-[D-(2-naphthylglycylamido)]-3-chloro-3-cephem-4-carboxylic acid; or a pharmaceutically-acceptable salt thereof.

18. 7-[D-(2-naphthylglycylamido)]-3-methoxy-3-cephem-4-carboxylic acid, or a pharmaceutically-acceptable salt thereof.

19. A process for preparing a compound of Formula (I), as claimed in any one of claims 1 to 18, which comprises

(A) acylating a compound of Formula (II):

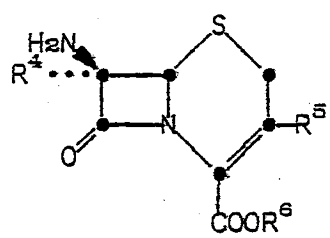

(II)

with an acylating agent of Formula (III):

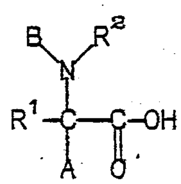

(III),

or an activated derivative thereof, in which A, B, $R^1$, $R^2$, $R^4$, $R^5$ and $R^6$ are as defined in claim 1, followed, optionally, by removal of any amino- or carboxyl-protecting groups present;

(B) deblocking of a protected acid of Formula (I) in which $R^6$ is a carboxy-protecting group to provide a compound of Formula (I) in which $R^6$ is hydrogen;

(C) removal of an amino-protecting group, $R^2$, from a compound of Formula (I) to provide a compound of Formula (I) in which $R^2$ is hydrogen;

(D) when it is desired to form a compound in which $R^2$ and $R^3$, when taken together form a group of the Formula:

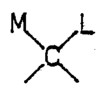 ,

reacting a compound of Formula (I), in which $R^2$ and $R^3$ both are hydrogen, with a ketone of the Formula:

M-C(O)-L

in which M and L are as defined in claim 1; or,

(E) reducing a compound of Formula (I) in which A and B are taken together to form a double bond and $R^2$ is hydroxy or methoxy, to produce a compound of Formula (I) in which A, B, and $R^2$ are hydrogen; and,

(F) if desired, salifying a compound of formula (I),

(G) or, if desired, converting a salt of a compound of Formula (I) to the free amine or acid.

20. A compound of Formula (I), or a pharmaceutically-acceptable salt thereof, as claimed in any one of claims 1 to 18, for use as an antibiotic in the chemotherapy of warm-blooded animals.

21. A pharmaceutical formulation which comprises as an active ingredient, a compound of Formula I, or a pharmaceutically-acceptable salt thereof, as claimed in any one of claims 1 to 18, associated with one or more pharmaceutically-acceptable carriers or vehicles therefor.

22. A tablet, capsule or oral suspension containing as its active ingredient, a compound of Formula (I), or a pharmaceutically-acceptable salt thereof, as claimed in any one of claims 1 to 18.

## CLAIMS

1. A process for preparing a compound of Formula (I):

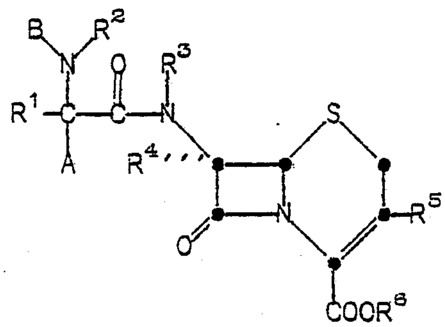

(I)

in which:

$R^1$ is

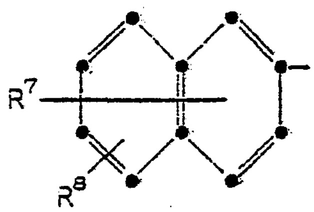

,

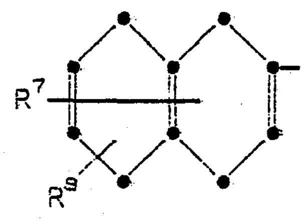

, or

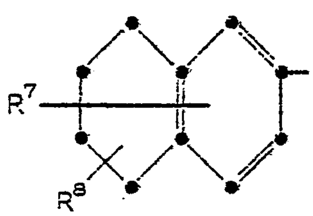

.

in which $R^7$ and $R^8$, independently, are hydrogen, halo, hydroxy, $C_1$-$C_4$ alkyl, $C_1$-$C_4$ alkoxy, nitro, amino, $C_1$-$C_4$ alkanoylamino, $C_1$-$C_4$ alkylsulfonylamino, or when $R^7$ and $R^8$ are taken together they form methylenedioxy;

A and B, both, are hydrogen, or taken together complete a double bond;

$R^2$ is hydrogen, an amino protecting group, hydroxy, or methoxy, and $R^3$ is hydrogen, or $R^2$ and $R^3$ taken together are

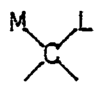 ,

where M and L, independently, are $C_1$-$C_4$ alkyl;

$R^4$ is hydrogen, methoxy or methylthio;

$R^5$ is hydrogen, methoxy, methyl, halo, or methoxymethyl;

$R^6$ is hydrogen, or a carboxy protecting group; provided that $R^2$ is hydroxy or methoxy only when A and B complete a double bond, and that A and B both are hydrogen when $R^3$ is other than hydrogen;

or a pharmaceutically-acceptable salt thereof, which comprises

(A) acylating a compound of Formula (II):

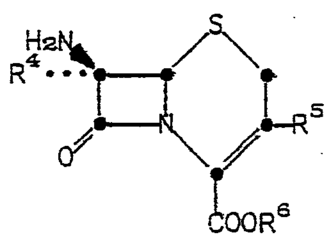 (II)

with an acylating agent of Formula (III):

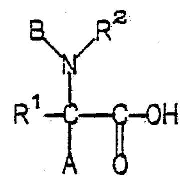

(III),

or an activated derivative thereof, in which A, B, $R^1$, $R^2$, $R^4$, $R^5$ and $R^6$ are as defined above, followed, optionally, by removal of any amino- or carboxyl-protecting groups present;

(B) deblocking of a protected acid of Formula (I) in which $R^6$ is a carboxy-protecting group to provide a compound of Formula (I) in which $R^6$ is hydrogen;

(C) removal of an amino-protecting group, $R^2$, from a compound of Formula (I) to provide a compound of Formula (I) in which $R^2$ is hydrogen;

(D) when it is desired to form a compound in which $R^2$ and $R^3$, when taken together form a group of the Formula:

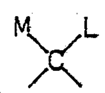

,

reacting a compound of Formula (I), in which $R^2$ and $R^3$ both are hydrogen, with a ketone of the Formula:

M-C(O)-L

in which M and L are as defined above; or,

(E) reducing a compound of Formula (I) in which A and B are taken together to form a double bond and $R^2$ is hydroxy or methoxy, to produce a compound of Formula (I) in which A, B, and $R^2$ are hydrogen; and,

(F) if desired, salifying a compound of formula (I),

(G) or, if desired, converting a salt of a compound of Formula (I) to the free amine or acid.

2. A process for preparing a compound of Formula (I), as claimed in claim 1, in which $R^2$ and $R^3$, when taken together, form

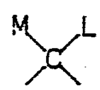 ;

or a pharmaceutically-acceptable salt thereof.

3. A process for preparing a compound of Formula (I), as claimed in claim 1, in which A and B, when taken together, complete a double bond and $R^2$ is methoxy; or a pharmaceutically-acceptable salt thereof.

4. A process for preparing a compound of Formula (I), as claimed in any one of claims 1 to 3, in which $R^1$ is

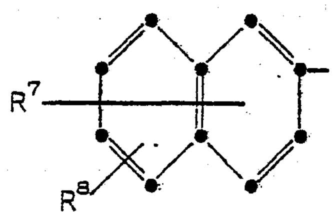

;

or a pharmaceutically-acceptable salt thereof.

5. A process for preparing a compound of Formula (I), as claimed in any one of claims 1 to 3 in which $R^1$ is

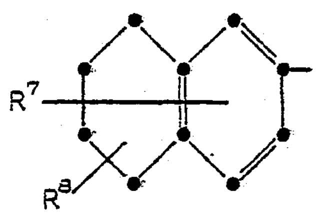

;

or a pharmaceutically-acceptable salt thereof.

6. A process for preparing a compound of Formula (I), as claimed in any one of claims 1 to 3 in which $R^1$ is

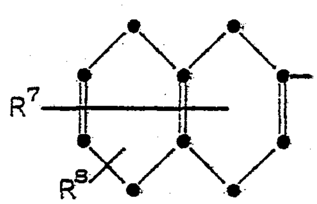

;

or a pharmaceutically-acceptable salt thereof.

7. A process for preparing a compound of Formula (I), as claimed in any one of claims 4 to 6, in which $R^7$ or $R^8$, independently, is hydrogen, halo, hydroxy, $C_1$-$C_4$ alkoxy, nitro or amino; or a pharmaceutically-acceptable salt thereof.

8. A process for preparing a compound of Formula (I), as claimed in claim 7, in which $R^7$ or $R^8$, independently, is hydrogen, halo, methoxy or hydroxy; or a pharmaceutically-acceptable salt thereof.

9. A process for preparing a compound of Formula (I), as claimed in claim 7 or 8, in which $R^7$ and $R^8$, both, are hydrogen; or a pharmaceutically-acceptable salt thereof.

10. A process for preparing a compound of Formula (I), as claimed in any one of claims 1 to 9, in which $R^5$ is methyl, chloro, hydrogen, methoxymethyl, or methoxy; or a pharmaceutically-acceptable salt thereof.

11. A process for preparing a compound of Formula (I), as claimed in claim 10, in which $R^5$ is methyl or chloro; or a pharmaceutically-acceptable salt thereof.

12. A process for preparing a compound of Formula (I), as claimed in any one of claims 1 to 11, in which $R^4$ is hydrogen; or a pharmaceutically-acceptable salt thereof.

13. A process for preparing a compound of Formula (I), as claimed in any one of claims 1 to 12, in which $R^6$ is hydrogen; or a pharmaceutically-acceptable salt thereof.

14. A process as claimed in claim 1 for preparing 7-[D-(2-naphthylglycylamido)]-3-methyl-3-cephem-4-carboxylic acid; or a pharmaceutically-acceptable salt thereof.

15. A process as claimed in claim 1 for preparing 7-[D-(2-naphthylglycylamido)]-3-methyl-3-cephem-4-carboxylic acid tetrahydrate.

16. A process as claimed in claim 1 for preparing 7-[D-(2-naphthylglycylamido)]-3-methyl-3-cephem-4-carboxylic acid hydrochloride monohydrate.

17. A process according to claim 1 for preparing 7-[D-(2-naphthylglycylamido)]-3-chloro-3-cephem-4-carboxylic acid; or a pharmaceutically-acceptable salt thereof.

18. A process according to claim 1 for preparing 7-[D-(2-naphthylglycylamido)]-3-methoxy-3-cephem-4-carboxylic acid, or a pharmaceutically-acceptable salt thereof.

19. A compound of Formula (I), or a pharmaceutically-acceptable salt thereof, whenever prepared by a process as claimed in any one of claims 1 to 18.